# EUROPEAN PATENT APPLICATION

(11) **EP 4 006 916 A1**
(43) Date of publication of application: **01.06.2022**
(21) Application number: 20843474.6
(22) Date of filing: 09.07.2020
(51) Int. Cl.: G16H 40/20, G16H 40/67

(54) **MOBILE BODY, INFORMATION PROCESSING DEVICE, AND INFORMATION PROCESSING METHOD**

(30) Priority: 23.07.2019 JP 2019135333
(71) Applicant: Sony Group Corporation, Tokyo 108-0075 (JP)
(72) Inventor: MIYAZAWA Katsuji, Tokyo 108-0075 (JP); SHINTANI Eiji, Tokyo 108-0075 (JP); YUMIBA Hiromu, Tokyo 108-0075 (JP); SUZUKI Aya, Tokyo 108-0075 (JP)
(74) Representative: Witte, Weller & Partner Patentanwälte mbB
(86) International application number: PCT/JP2020/026867
(87) International publication number: WO 2021/014987

(57) **Abstract**

The present technology relates to a moving object, an information processing device, and an information processing method capable of facilitating a patient to receive telemedicine. A moving object includes a movement control unit configured to control movement based on a patient demand; a communication control unit configured to control communication with a medical practitioner terminal which is an information processing terminal of a medical practitioner executing telemedicine; and a medical control unit configured to control the telemedicine with the information processing terminal. The present technology can be applied to, for example, a system that executes telemedicine.

## Description

### [Technical Field]

The present technology relates to a moving object, an information processing device, an information processing method, and more particularly, to a moving object, an information processing device, and an information processing method appropriate for telemedicine.

### [Background Art]

In the related art, systems that match homecare patients with nurses have been proposed (for example, see PTL 1).

In recent years, telemedicine has been studied so that examination or treatment can be performed remotely for patients staying at home even though doctors or nurses do not visit patient's homes.

### [Citation List]

### [Patent Literature]

[PTL 1] JP 2018-45668 A

### [Summary]

### [Technical Problem]

However, depending on content of telemedicine, patients cannot receive telemedicine in some cases when the patients do not have the necessary devices.

The present technology has been devised in view of such circumstances so that patients can easily receive telemedicine.

### [Solution to Problem]

According to a first aspect of the present technology, a moving object includes: a movement control unit configured to control movement based on a patient demand; a communication control unit configured to control communication with a medical practitioner terminal which is an information processing terminal of a medical practitioner executing telemedicine; and a medical control unit configured to control the telemedicine with the medical practitioner terminal.

According to the first aspect of the present technology, an information processing method causes a moving object to perform: controlling movement based on a patient demand; controlling communication with a medical practitioner terminal which is an information processing terminal of a medical practitioner executing telemedicine; and controlling the telemedicine with the medical practitioner terminal.

According to a second aspect of the present technology, an information processing device includes: a selection unit configured to select a medical practitioner introduced to a patient; and a communication control unit configured to control transmission of information regarding the medical practitioner introduced to the patient to a moving object used by the patient.

According to the first aspect of the present technology, movement is controlled based on a patient demand, communication with a medical practitioner terminal which is an information processing terminal of a medical practitioner executing telemedicine is controlled, and the telemedicine with the medical practitioner terminal is controlled.

According to the second aspect of the present technology, a medical practitioner introduced to a patient is selected; and transmission of information regarding the medical practitioner introduced to the patient to a moving object used by the patient is controlled.

### [Brief Description of Drawings]

[Fig. 1]

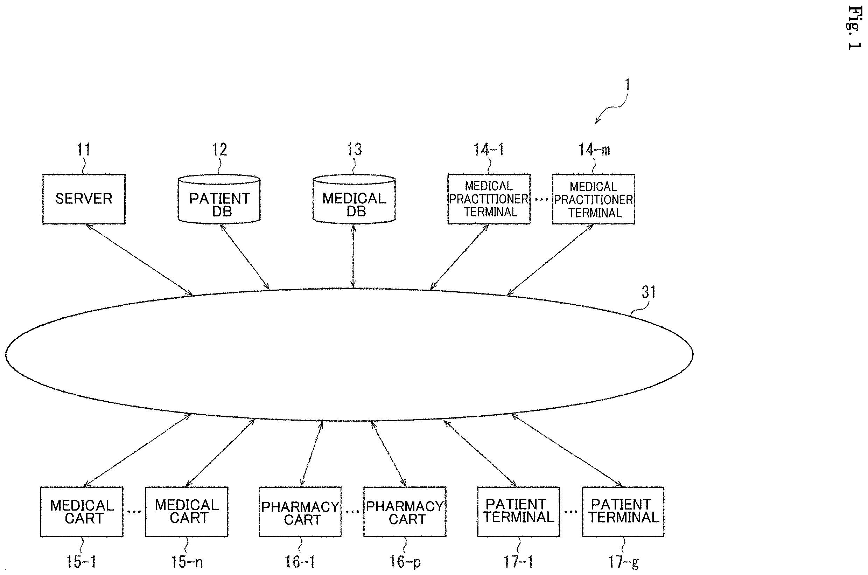

   Fig. 1 is a block diagram illustrating an exemplary configuration of a telemedicine system to which the present technology is applied.
[Fig. 2]

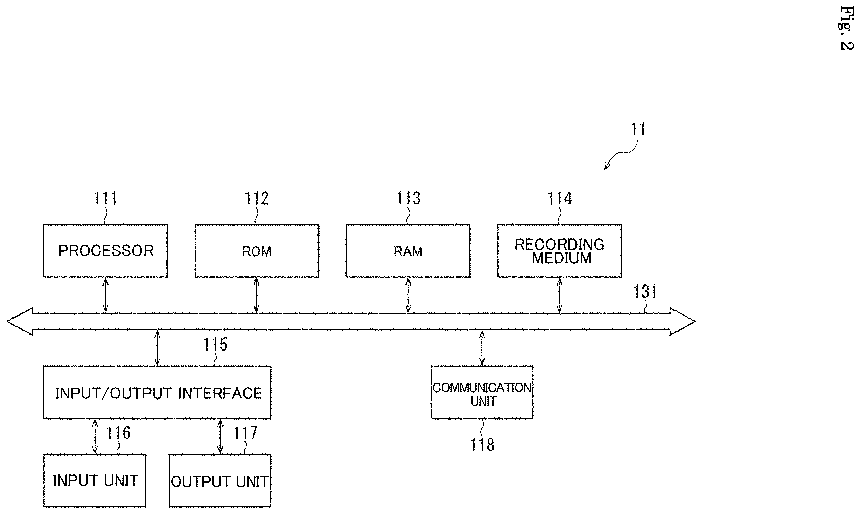

   Fig. 2 is a block diagram illustrating an exemplary configuration of a server.
[Fig. 3]

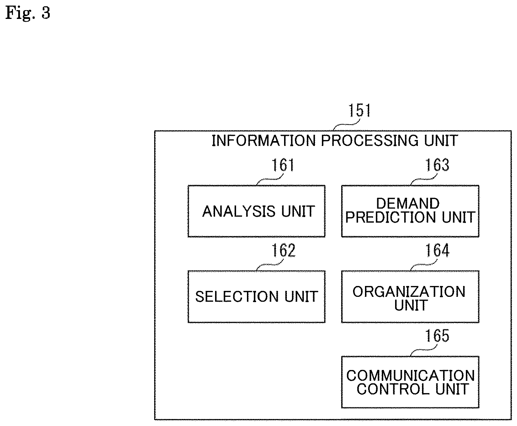

   Fig. 3 is a block diagram illustrating an exemplary configuration of an information processing unit realized by a processor of the server.
[Fig. 4]

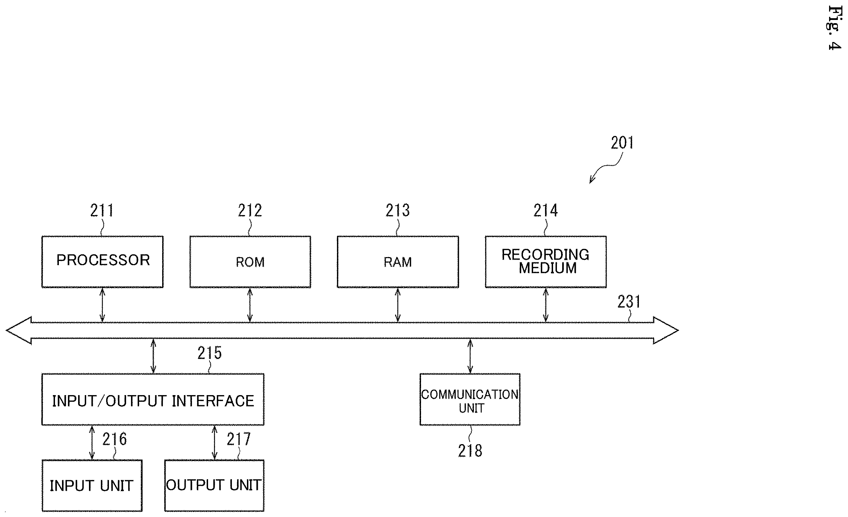

   Fig. 4 is a block diagram illustrating an exemplary configuration of an information processing terminal.
[Fig. 5]

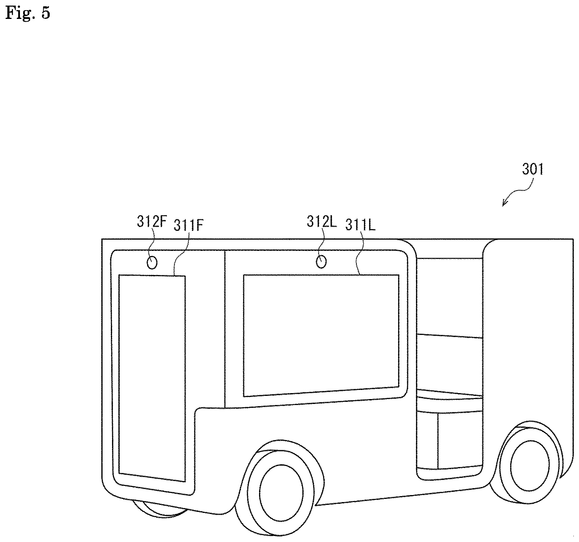

   Fig. 5 is a diagram illustrating an exemplary configuration of the exterior appearance of a vehicle configuring a medical cart and a pharmacy cart.
[Fig. 6]

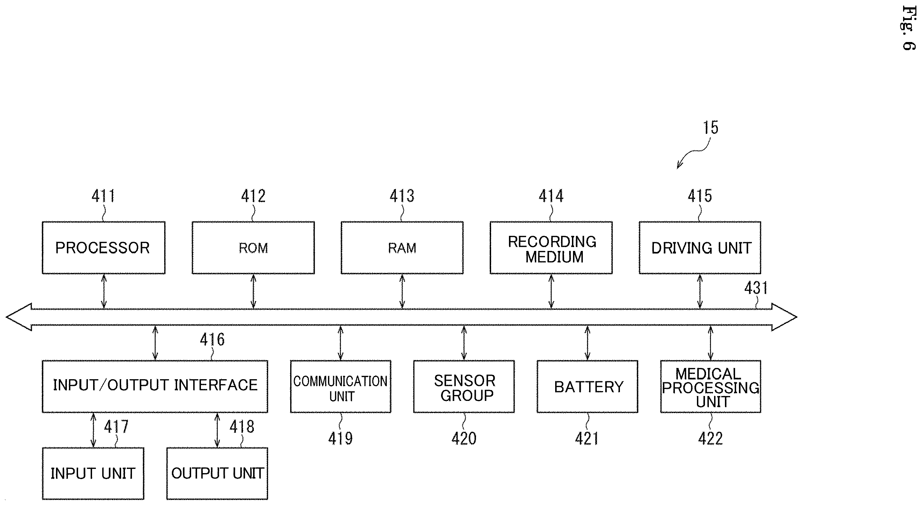

   Fig. 6 is a block diagram illustrating an exemplary configuration of the medical cart.
[Fig. 7]

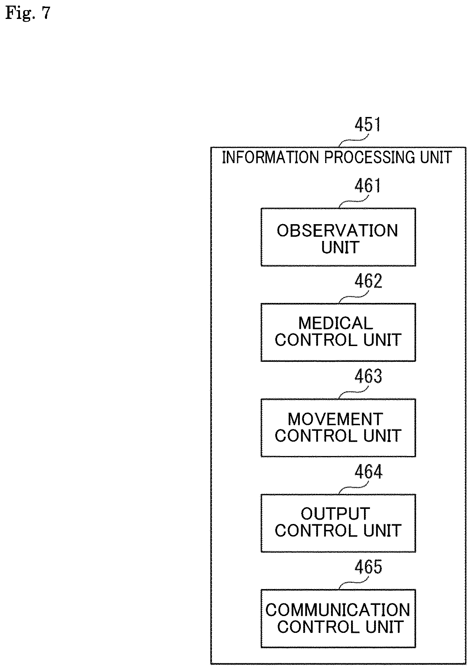

   Fig. 7 is a block diagram illustrating an exemplary configuration of an information processing unit realized by a processor of the medical cart.
[Fig. 8]

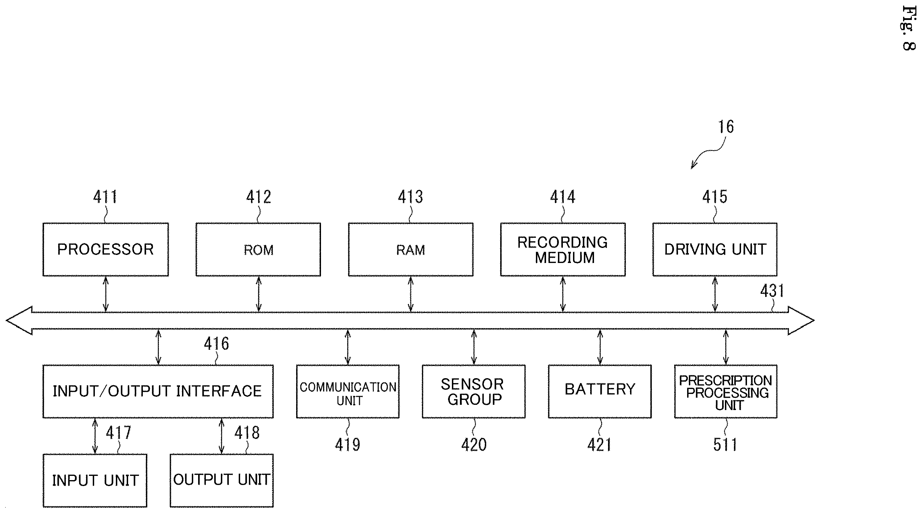

   Fig. 8 is a block diagram illustrating an exemplary configuration of the pharmacy cart.
[Fig. 9]

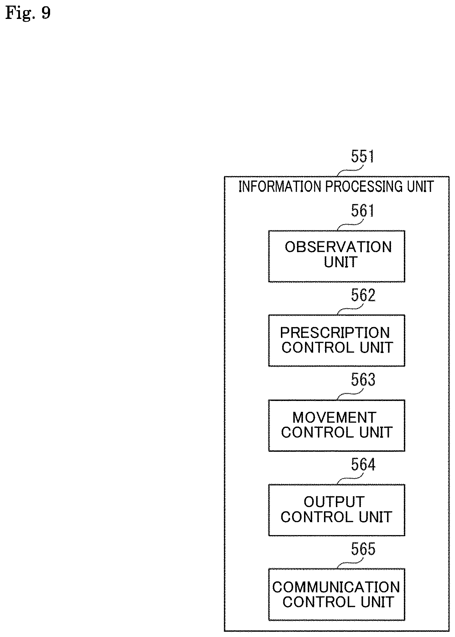

   Fig. 9 is a block diagram illustrating an exemplary configuration of an information processing unit realized by a processor of the pharmacy cart.
[Fig. 10]

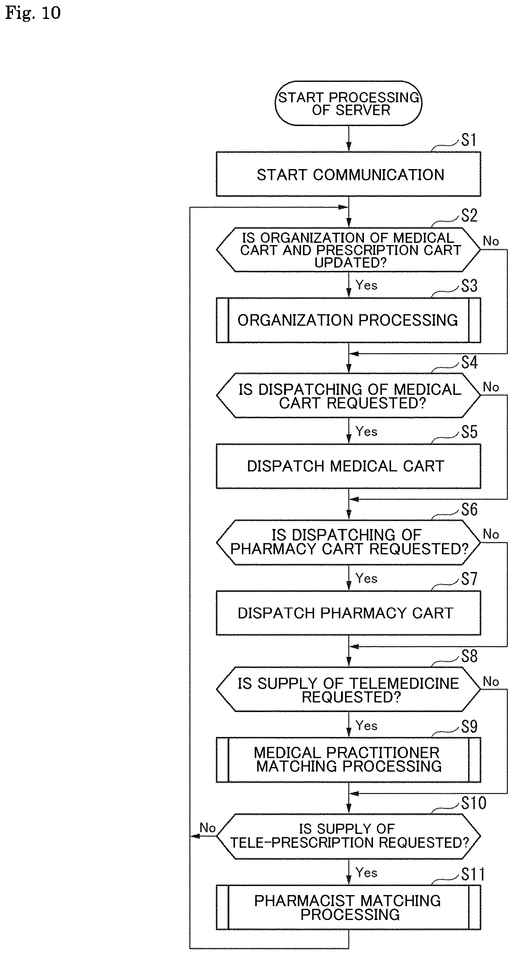

   Fig. 10 is a flowchart illustrating processing of the server.
[Fig. 11]

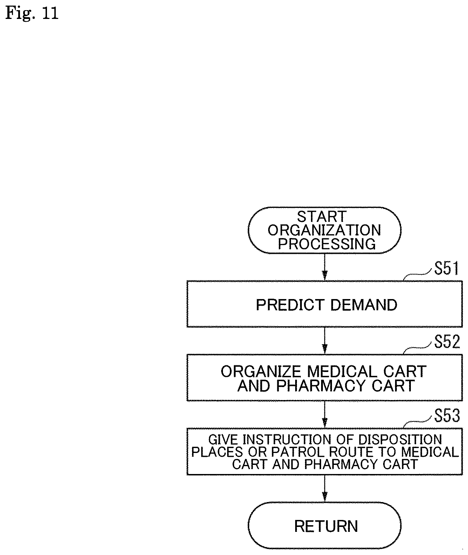

   Fig. 11 is a flowchart illustrating details of organization processing.
[Fig. 12]

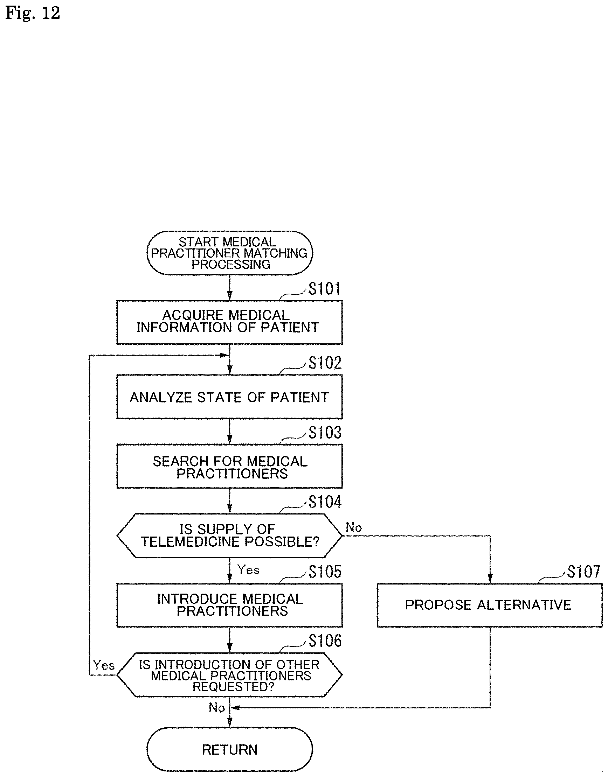

   Fig. 12 is a flowchart illustrating details of medical practitioner matching processing.
[Fig. 13]

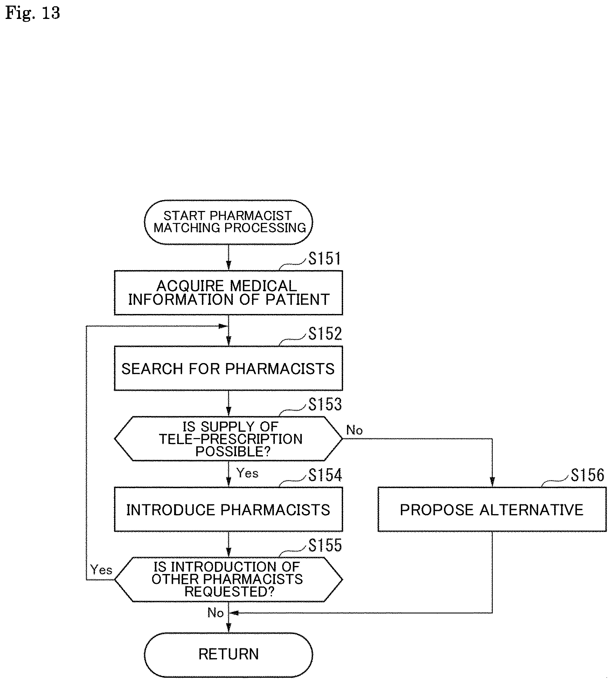

   Fig. 13 is a flowchart illustrating details of pharmacist matching processing.
[Fig. 14]

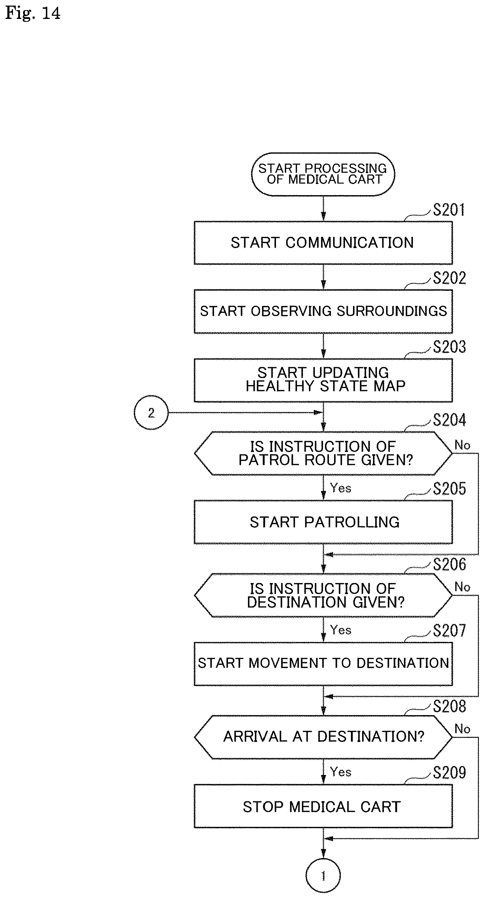

   Fig. 14 is a flowchart illustrating processing of the medical cart.
[Fig. 15]

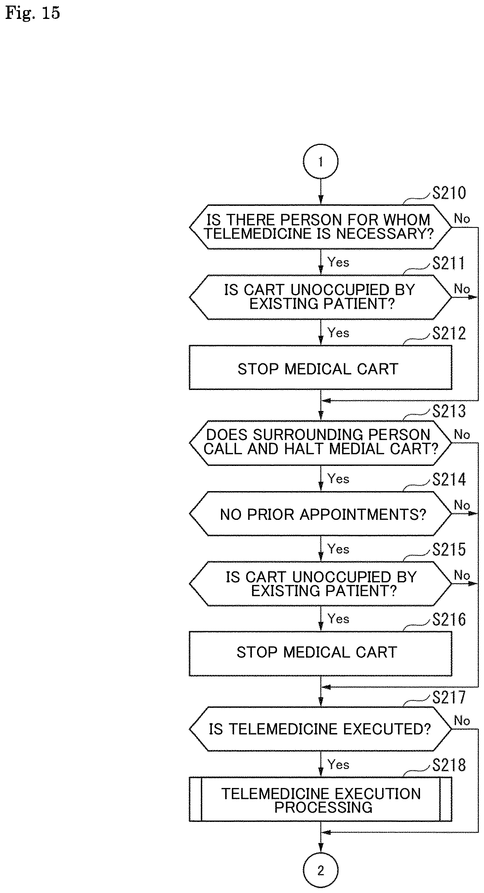

   Fig. 15 is a flowchart illustrating processing of the medical cart.
[Fig. 16]

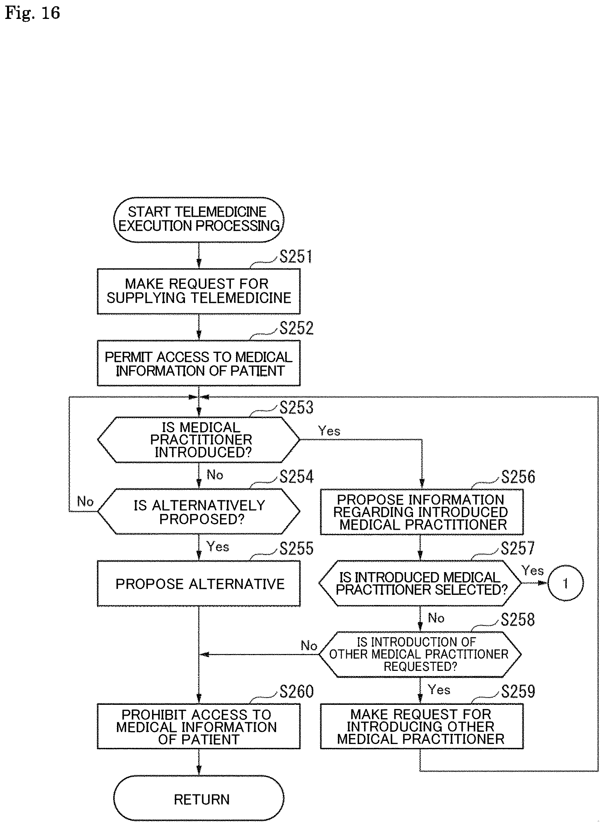

   Fig. 16 is a flowchart illustrating details of telemedicine execution processing.
[Fig. 17]

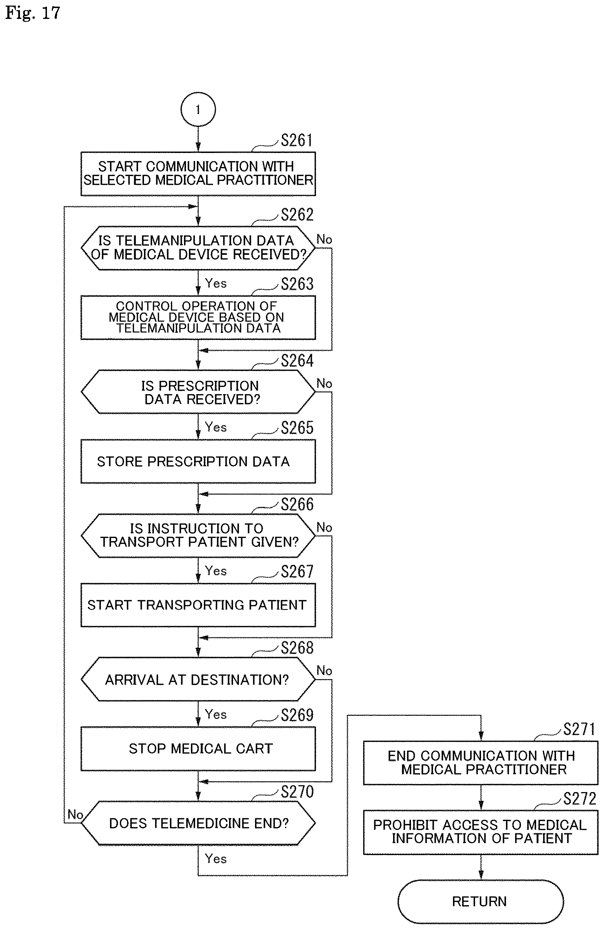

   Fig. 17 is a flowchart illustrating details of the telemedicine execution processing.
[Fig. 18]

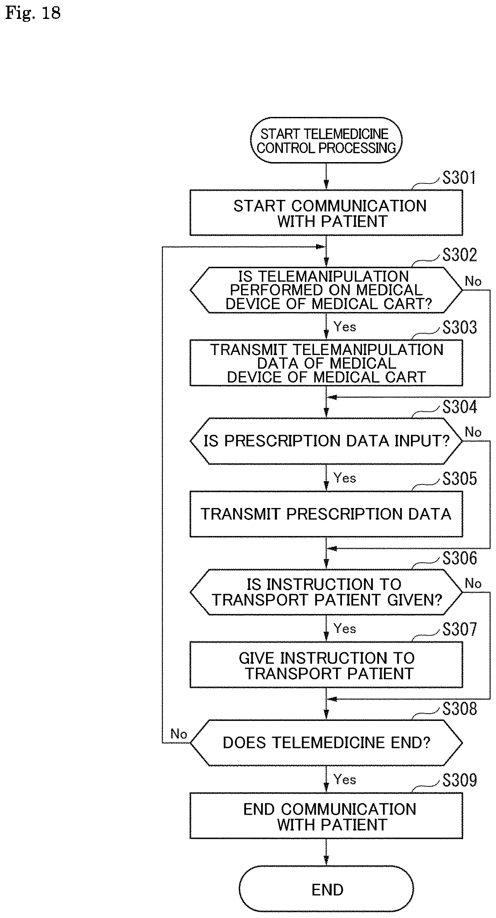

   Fig. 18 is a flowchart illustrating telemedicine control processing.
[Fig. 19]

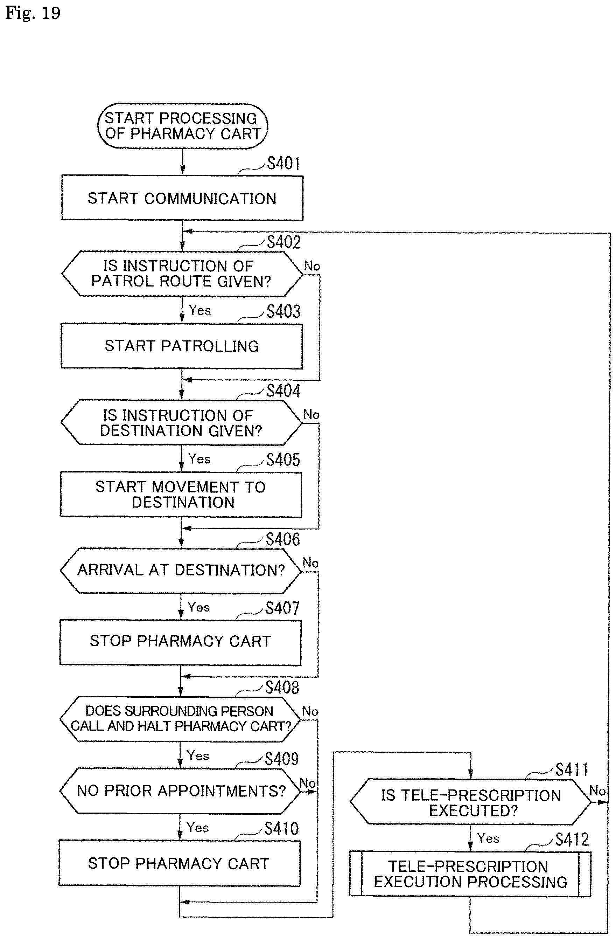

   Fig. 19 is a flowchart illustrating processing of the pharmacy cart.
[Fig. 20]

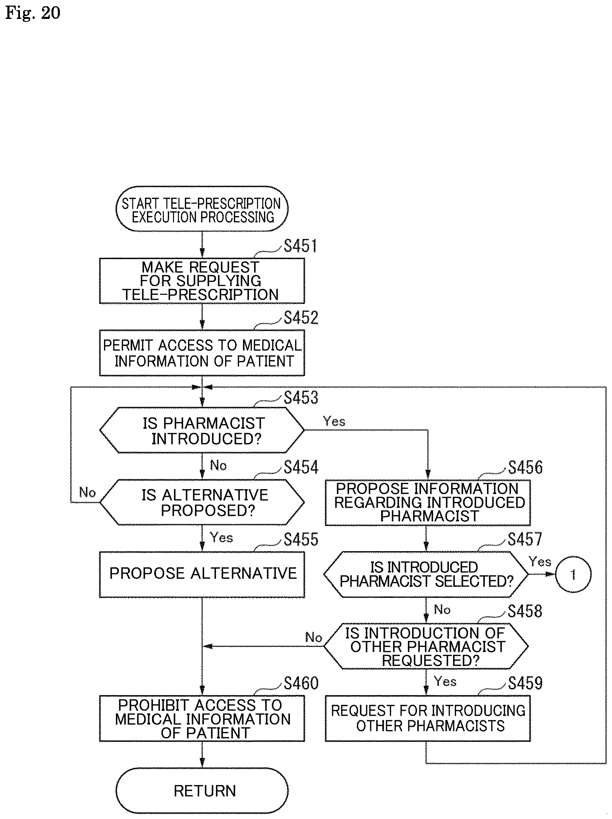

   Fig. 20 is a flowchart illustrating details of tele-prescription execution processing.
[Fig. 21]

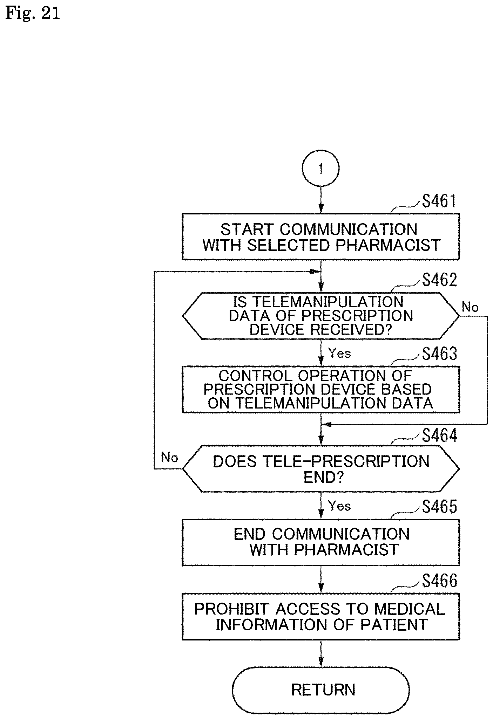

   Fig. 21 is a flowchart illustrating details of the tele-prescription execution processing.
[Fig. 22]

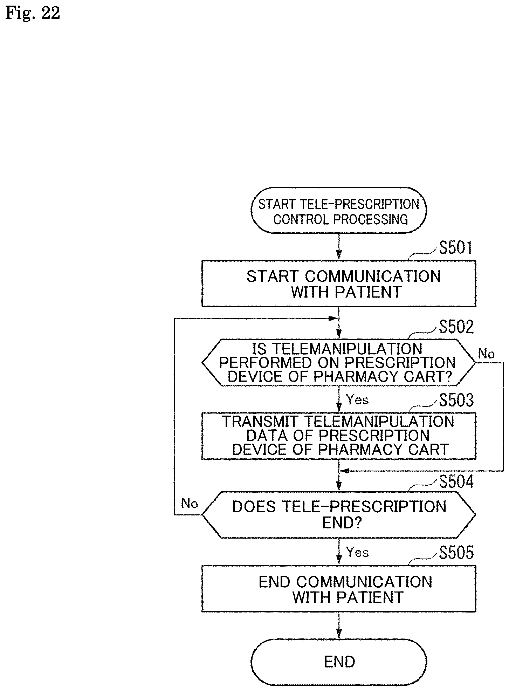

   Fig. 22 is a flowchart illustrating tele-prescription control processing.
[Fig. 23]

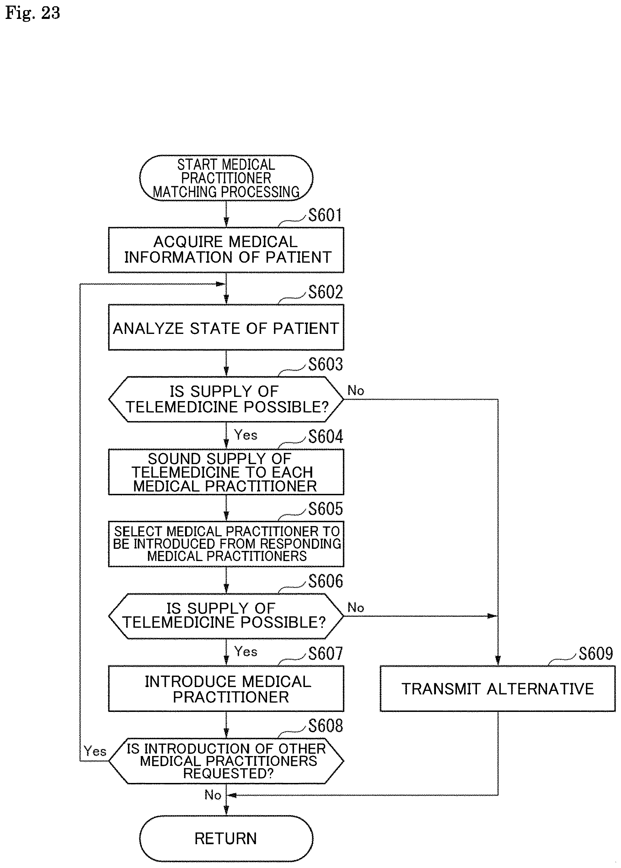

   Fig. 23 is a flowchart illustrating a modified example of the medical practitioner matching processing.
[Fig. 24]

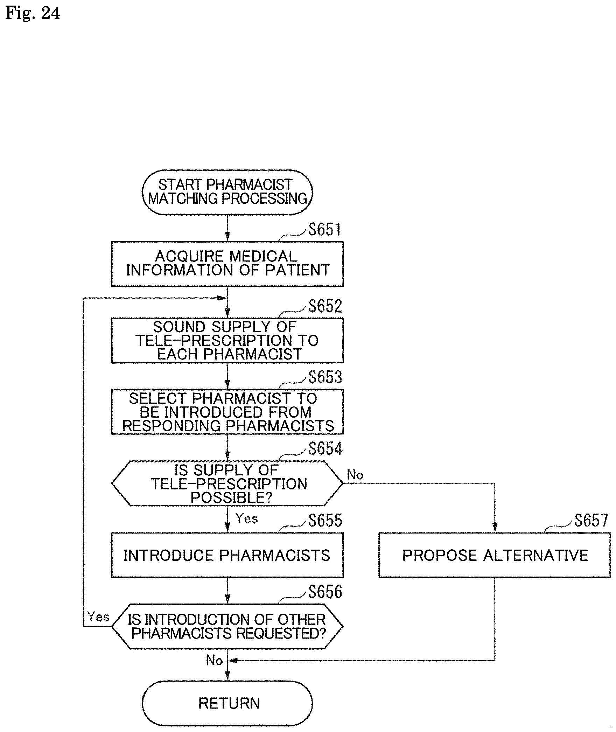

   Fig. 24 is a flowchart illustrating a modified example of the pharmacist matching processing.
[Fig. 25]

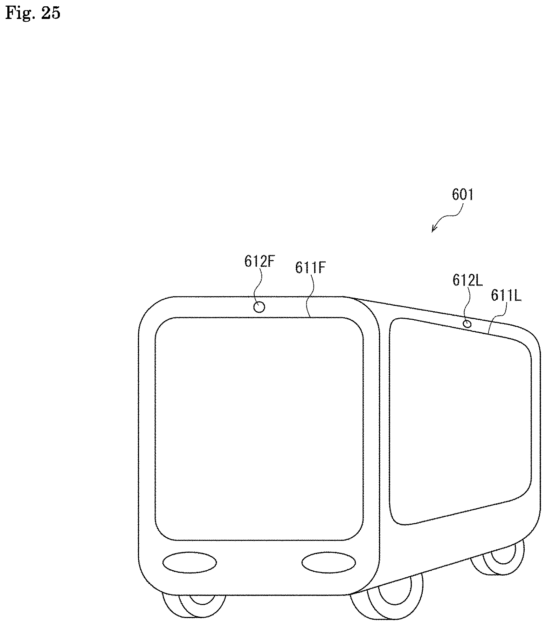

   Fig. 25 is a diagram illustrating a modified example of the exterior appearance of a vehicle configuring a medical cart and a pharmacy cart.

### [Description of Embodiments]

Hereinafter, modes for carrying out the present technology will be described. The description will be made in the following order.
1. Embodiment
2. Modified Examples
3. Others

### «1. Embodiment»

First, an embodiment of the present technology will be described with reference to Figs. 1 to 22.

### <Exemplary Configuration of Telemedicine System 1>

Fig. 1 is a block diagram illustrating an embodiment of a telemedicine system 1 to which the present technology is applied.

The telemedicine system 1 is a system that provides a telemedicine service. Specifically, the telemedicine system 1 is a system in which medical practitioners (medical workers) execute telemedicine and tele-prescription for patients.

The telemedicine is, for example, a service in which medical practitioners remotely execute various medical actions using a communication technology. The medical actions which can be supplied by the telemedicine system 1 are not necessarily limited to medical actions regulated in laws or the like and include various actions contributing to health improvement, medical, healthcare, and nursing. Specifically, for example, medical examination, medical treatment, medical consultation, health consultation, medication instruction, medicine prescription, counseling, and therapy are included.

The tele-prescription is, for example, a service in which a pharmacist among the medical practitioners remotely dispenses medicine and supplies the medicine to a patient or gives a medication instruction.

Further, in the present technology, medical practitioners are people who execute telemedicine or tele-prescription for patients and are not necessarily limited to people who have national qualifications for doctors, dentists, pharmacists, nurses, public health nurses, and the like. The medical practitioners may not necessarily work for specific medical organizations such as hospitals or drug stores and may be, for example, freelancers or people pm alert at their homes.

As described above, the medical practitioners include pharmacists. When pharmacists are particularly distinguished from other medical practitioners, the term, pharmacists, are used instead of medical practitioners.

In the present technology, patients are people to whom services for telemedicine or tele-prescription are supplied and may be healthy people and do not necessarily have to be those suffering from diseases, injuries, or the like requiring treatment.

The telemedicine system 1 includes a server 11, a patient database (DB) 12, a medical database (DB) 13, medical practitioner terminals 14-1 to 14-m, medical carts 15-1 to 15-n, pharmacy carts 16-1 to 16-p, patient terminals 17-1 to 17-q, and a network 31. The server 11, the patient database (DB) 12, the medical database (DB) 13, the medical practitioner terminals 14-1 to 14-m, the medical carts 15-1 to 15-n, the pharmacy carts 16-1 to 16-p, and the patient terminals 17-1 to 17-q are connected to each other via the network 31 so that mutual communication is possible.

Hereinafter, when it is not necessary to distinguish the medical practitioner terminals 14-1 to 14-m, the medical carts 15-1 to 15-n, the pharmacy carts 16-1 to 16-p, and the patient terminals 17-1 to 17-q from each other, these are simply referred to as the medical practitioner terminals 14, the medical carts 15, the pharmacy carts 16, and the patient terminals 17, respectively.

The server 11 controls the whole telemedicine system 1. For example, the server 11 predicts a patient demand and organizes the medical carts 15 and the pharmacy carts 16. For example, the server 11 matches medical practitioners with patient based on the patient DB 12 and the medical DB 13, and the like and performs processing for appropriately introducing the medical practitioners to the patients.

The patient DB 12 is a database that stores information regarding patients using the telemedicine system 1. For example, the patient DB 12 stores personal information and medical information regarding patients.

The personal information regarding a patient includes, for example, a name, an age, a gender, a nationality, an address, a contact address, and an insurance card number, etc.

The medical information regarding a patient includes, for example, a clinical card, a case history, a surgery history, a hospitalization history, a medication history, health examination results, and allergy information. The case history, the surgery history, the hospitalization history, and the medication history may be included in the clinical card.

The medical DB 13 stores medical practitioner information regarding medical practitioners who can execute telemedicine and tele-medication using the telemedicine system 1 and medical organization information regarding medical organizations which can be used by patients.

The medical practitioner information includes, for example, a facial photo, a profile, a field of specialization (for example, a medical department or the like), a career, and a schedule of each medical practitioner. The profile of the medical practitioner includes, for example, a name, a gender, a nationality, a contact address, an affiliated medical organization, and available languages. The information regarding a career of a medical practitioner includes, for example, an employment record, the number of past surgical operations, published works, related newspapers, magazines, articles of web sites, and theses.

The medical organization information includes, for example, a name, an address, a contact address, reception hours, medical examination hours, information regarding affiliated medical practitioners, an applicable medical field (for example, a medical department or the like), a size, and facilities of each medical organization.

The medical practitioner terminal 14 is an information processing terminal used for each medical practitioner to supply telemedicine or tele-prescription. The type of medical practitioner terminal 14 does not particularly matter as long as the medical practitioner terminal 14 can supply telemedicine or tele-prescription. For example, the medical practitioner terminal 14 is configured by a personal computer (PC), a tablet terminal, a smartphone, a mobile phone, or the like.

For example, one medical practitioner terminal 14 may be shared by a plurality of medical practitioners or a plurality of medical practitioner terminals 14 may be used by one medical practitioner. A place where the medical practitioner terminal 14 is installed does not particularly matter. For example, the medical practitioner terminal 14 may be installed in a medical organization such as a hospital or a pharmacy, may be installed in a home of a medical practitioner, or may be carried by a medical practitioner.

The medical cart 15 includes a medical device used for tele-medicine and is used for each patient to receive tele-medicine.

The pharmacy cart 16 includes a prescription device used for tele-prescription and is used for each patient to receive a tele-prescription.

The medical cart 15 and the pharmacy cart 16 can be moved in an unmanned manner by automatic driving or telemanipulation (remote driving).

The patient terminal 17 is, for example, an information processing terminal used to request dispatching of the medical cart 15 and the pharmacy cart 16. One patient terminal 17 may be shared by a plurality of patients or the plurality of patient terminals 17 may be shared by one patient. The type of patient terminal 17 does not particularly matter as long as the patient terminal can be used to request dispatching of the medical cart 15 and the pharmacy cart 16. For example, the patient terminal 17 is configured by a personal computer (PC), a tablet terminal, a smartphone, a mobile phone, or the like.

### <Exemplary Configuration of Server 11>

Fig. 2 is a block diagram illustrating an exemplary configuration of the server 11 of the telemedicine system 1 in Fig. 1.

The server 11 includes a processor 111, a read-only memory (ROM) 112, a random access memory (RAM) 113, a recording medium 114, an input/output interface 115, an input unit 116, an output unit 117, and a communication unit 118. The processor 111, the ROM 112, the RAM 113, the recording medium 114, the input/output interface 115, and the communication unit 118 are connected to each other via a bus 131.

The processor 111 includes, for example, one or more processors configured with an arithmetic circuit such as a micro processing unit (MPU) and various processing circuits. The processor 111 controls the whole server 11.

The ROM 112 stores, for example, control data such as arithmetic parameters and a program used by the processor 111.

The RAM 113 temporarily stores, for example, a program executed by the processor 111 and data used for processing of the processor 111.

The recording medium 114 is configured by a magnetic disk, an optical disc, a magneto-optical disc, or a semiconductor memory. The recording medium 114 stores various programs, applications, data, and the like. The recording medium 114 may be detachably mounted on the server 11.

The input/output interface 115 connects, for example, the input unit 116 or the output unit 117. The input/output interface 115 is configured by, for example, a USB terminal, a DVI terminal, an HDMI (registered trademark) terminal, or any of various processing circuits.

The input unit 116 includes, for example, various input devices and is used to input various kinds of data, instructions, or the like. The type and number of input devices included in the input unit 116 is not particularly limited. A touch panel, a button, a switch, a microphone, and the like is used as necessary.

The output unit 117 includes, for example, various output devices capable of outputting one or more of visual information, auditory information, and tactile information. The type and number of output devices included in the output unit 117 are not particularly limited. A display device, a speaker, a light-emitting device, a vibration element, and the like are used as necessary.

The communication unit 118 includes one or more communication devices and performs communication with other devices of the telemedicine system 1 via the network 31 in conformity with a predetermined communication scheme. Any wired or wireless communication scheme can be adopted to the communication unit 118. The communication unit 118 may correspond to a plurality of communication schemes.

In the following description of the server 11, the input/output interface 115 and the bus 131 will not be described. For example, when the processor 111 and the output unit 117 transmit and receive data via the bus 131 and the input/output interface 115, mention of the bus 131 and the input/output interface 115 will be omitted. When the processor 111 and the output unit 117 mutually transmit and receive data, the processor 111 and the output unit 117 will be described.

### <Exemplary Configuration of Information Processing Unit 151>

Fig. 3 illustrates an exemplary configuration of an information processing unit 151 which is a part of a function realized by allowing the processor 111 of the server 11 to execute a predetermined control program.

The information processing unit 151 includes an analysis unit 161, a selection unit 162, a demand prediction unit 163, an organization unit 164, and a communication control unit 165.

The analysis unit 161 analyzes a patient state based on information received from the patient DB 12, the medical DB 13, and the medical cart 15, information received from the pharmacy cart 16, and information received from the patient terminal 17. The analysis unit 161 selects a telemedicine action necessary for a patient based on an analysis result of the patient state. Further, the analysis unit 161 determines whether telemedicine and tele-prescription can be supplied to a patient. When the analysis unit 161 determines that the telemedicine and the tele-prescription cannot be supplied, an alternative is generated and alternative information for presenting the generated alternative is transmitted to the medical cart 15 and the pharmacy cart 16 via the network 31.

The selection unit 162 performs processing for matching the patient state with a medical practitioner based on at least one of the patient DB 12, the medical DB 13, a facility of the medical cart 15, a facility of the pharmacy cart 16, a schedule of each medical practitioner, a patient state, and a desired condition, and selects a medical practitioner introduced to a patient. The selection unit 162 transmits information indicating the introduced medical practitioner to the medical cart 15 or the pharmacy cart 16 used by each patient. For example, the selection unit 162 transmits information regarding medical practitioners who are introduced to patients and execute telemedicine to the medical cart 15 with which a patient receives the telemedicine. For example, the selection unit 162 transmits information regarding pharmacists who are medical practitioners who are introduced to patients and execute tele-prescription to the medical cart 15 with which a patient receives the tele-prescription.

The demand prediction unit 163 collects various kinds of information from each medical practitioner terminal 14, each medical cart 15, each pharmacy cart 16, another server (not illustrated), and the like via the network 31 and the communication unit 118 and predicts a patient demand for the medical cart 15 and the pharmacy cart 16 based on the collected information.

The organization unit 164 organizes the medical cart 15 and the pharmacy cart 16 based on at least one of a prediction result of the patient demand, a position and state of each medical cart 15, a position and state of each pharmacy cart 16, a request of a patient received from the patient terminal 17 (a demand from patients), and the like. For example, the organization unit 164 gives an instruction for a patrol route, dispatching, or the like to each medical cart 15 and each pharmacy cart 16.

The communication control unit 165 controls communication processing of the communication unit 118.

### <Exemplary Configuration of Information Processing Device 201>

Fig. 4 illustrates an exemplary configuration of the information processing device 201 which can configure each medical practitioner terminal 14 and each patient terminal 17 of the telemedicine system 1. In the drawing, reference numerals with the same two low-order digits are given to corresponding units in the server 11 in Fig. 2.

The information processing device 201 has the same configuration as the server 11, and thus description thereof will be omitted.

### <Exemplary Configuration of Vehicle 301>

Fig. 5 illustrates an exemplary configuration of the exterior appearance of a vehicle 301 that configures the medical cart 15 and the pharmacy cart 16. Fig. 5 illustrates an exemplary configuration of the exterior appearance when the vehicle 301 is viewed diagonally in front and to the left.

The vehicle 301 is a moving object that can move in an unmanned manner by automatic driving or telemanipulation.

When the vehicle 301 is moving, people may be on board. The vehicle 301 may be driven by a driver.

As the vehicle 301, for example, an electric automobile which moves by a motor such as an electric cart is exemplified. The vehicle 301 according to the embodiment is not limited to an electric automobile.

A display unit 311F, a display unit 311L, a display unit 311R (not illustrated), and a display unit 311B (not illustrated) are provided on the front surface, the left side surface, the right side surface, and the back surface of the vehicle 301, respectively. The display unit 311F to the display unit 311B are configured by, for example, a thin display device such as a liquid crystal display (LCD) or an organic EL panel.

A camera 312F, a camera 312L, a camera 312R (not illustrated), and a camera 312B (not illustrated) are provided on the front surface, the left side surface, the right side surface, and the back surface of the vehicle 301. The camera 312F mainly images the side in front of the vehicle 301. The camera 312L mainly images the side to the left of the vehicle 301. The camera 312R mainly images the right side of the vehicle 301. The camera 312B mainly images the back side of the vehicle 301.

Types or specifications of the cameras 312F to 312B are not particularly limited. For example, the cameras 312F to 312B may be configured by stereo cameras.

Inside the vehicle 301, there is a space in which a patient can receive telemedicine or tele-prescription. In the space, for example, a camera that images a patient, a microphone that collects a sound from the patient, an information processing terminal that performs telecommunication or information processing, a desk, a chair, and a medical device, a prescription device, and the like are provided. The space is preferably a closed space distant from the outside to protect security and privacy of a patient.

### <Exemplary Configuration of Medical Cart 15>

Fig. 6 illustrates an exemplary configuration of the medical cart 15 of the telemedicine system 1 of Fig. 1 configured by the vehicle 301 of Fig. 5. The medical cart 15 includes a processor 411, a ROM 412, a RAM 413, a recording medium 414, a driving unit 415, an input/output interface 416, an input unit 417, an output unit 418, a communication unit 419, a sensor group 420, a battery 421, a medical processing unit 422, and a bus 431. The processor 411, the ROM 412, the RAM 413, the recording medium 414, the driving unit 415, the input/output interface 416, the communication unit 419, the sensor group 420, the battery 421, and the medical processing unit 422 are connected to each other via a bus 431.

The processor 411 includes, for example, one or more processors configured with an arithmetic circuit such as an MPU and various processing circuits. The processor 411 controls the whole medical cart 15.

The ROM 412 stores, for example, control data such as arithmetic parameters and a program used by the processor 411.

The RAM 413 temporarily stores, for example, a program executed by the processor 411 and data used for processing of the processor 411.

The recording medium 414 is configured by a magnetic disk, an optical disc, a magneto-optical disc, or a semiconductor memory. The recording medium 414 stores various programs, applications, data, and the like. The recording medium 414 may be detachably mounted on the medical cart 15.

The driving unit 415 includes, for example, various devices (or mechanisms) related to movement of the medical cart 15, such as a power source such as a motor, wheels, transmission devices transmitting power generated by the power source to the wheels, a brake mechanism.

The input/output interface 416 connects, for example, the input unit 417 or the output unit 418. The input/output interface 416 is configured by, for example, a USB terminal, a DVI terminal, an HDMI (registered trademark) terminal, or any of various processing circuits.

The input unit 417 includes, for example, various input devices and is used to input driving and manipulation of the medical cart 15, various kinds of data, instructions, or the like. The type and number of input devices of the input unit 417 is not particularly limited. For example, the input unit 417 includes a game controller including a direction key, a button, and a stick-shaped device, controllers including a steering wheel, a shift lever, an acceleration pedal, and a brake pedal, a touch panel, a button, and a switch.

The output unit 418 includes, for example, various output devices capable of outputting one or more of visual information, auditory information, tactile information. For example, the output unit 418 includes a display device that displays an image inside or outside of the vehicle 301, such as the display units 311F to 311B. The type and number of output devices included in the output unit 418 are not particularly limited. A display device, a speaker, a light-emitting device, a vibration element, and the like are used as necessary.

The communication unit 419 includes one or more communication devices and performs communication with other devices of the telemedicine system 1 via the network 31 in conformity with a predetermined communication scheme. Any wired or wireless communication scheme can be adopted to the communication unit 419. The communication unit 419 may correspond to a plurality of communication schemes.

The sensor group 420 includes various sensors detecting a state or the like of the medical cart 15. For example, the sensor group 420 includes a gyro sensor, an acceleration sensor, an inertial measurement unit (IMU), and a sensor detecting a motor rotation speed, a rotation speed of a wheel, or the like.

For example, the sensor group 420 includes various sensors detecting external information. For example, the sensor group 420 includes imaging devices such as the above-described cameras 312F to 312B. Examples of the imaging devices include a time of flight (ToF) camera, a stereo camera, a monocular camera, and an infrared camera. For example, the sensor group 420 includes an environmental sensor that detects weather, climate, or the like and a circumferential information detection sensor that detects surrounding objects. The environmental sensor is configured by, for example, a raindrop sensor, a fog sensor, a sunshine sensor, a snow sensor, or the like. The circumferential information detection sensor is configured by, for example, an ultrasonic sensor, a radar, a light detection and ranging laser imaging detection and ranging (LiDAR), a sonar, or the like.

Further, for example, the sensor group 420 includes various positional sensors that detect a current position. For example, the sensor group 420 includes a global navigation satellite system (GNSS) receiver that receives a GNSS signal from a GNSS satellite.

For example, the sensor group 420 includes various sensors that detect internal information of the medical cart 15. For example, the sensor group 420 includes an imaging device that images the inside of the medical cart 15 and a microphone that collects an internal sound of the medical cart 15.

The communication unit 419 includes one or more communication devices and performs communication with other devices of the telemedicine system 1 via the network 31 in conformity with a predetermined communication scheme. Any wired or wireless communication scheme can be adopted to the communication unit 419. The communication unit 419 may correspond to a plurality of communication schemes.

The battery 421 is an internal power source included in the medical cart 15. Each device driven with power in the medical cart 15 is driven by, for example, power supplied from the battery 421. The medical cart 15 can also be driven by power supplied from an external power source.

The medical processing unit 422 include various medical device and executes various medical actions. For example, the medical processing unit 422 includes a medical robot that remotely executes a surgical operation, an examination, treatments, measurement, and the like. For example, the medical processing unit 422 includes measurement devices that measure a blood pressure, a pulse, a body temperature, a height, a weight, a chest state, and a physical status of a patient.

The medical devices included in the medical processing unit 422 may not necessarily be all able to be remotely manipulated. For example, a clinical thermometer, a blood pressure meter, an injection, or the like may be manipulated by a nurse, a patient, or the like in person.

The types of medical devices included in the medical processing unit 422 may be different for each medical cart 15.

### <Exemplary Configuration of Information Processing Unit 451>

Fig. 7 illustrates an exemplary configuration of an information processing unit 451 which is a part of a function realized by allowing the processor 411 of the medical cart 15 to execute a predetermined control program.

The information processing unit 451 includes an observation unit 461, a medical control unit 462, a movement control unit 463, an output control unit 464, and a communication control unit 465.

The observation unit 461 observes the inside and the surroundings of the medical cart 15 based on sensor data supplied from the sensor group 420. For example, the observation unit 461 observes a patient inside the medical cart 15. For example, the observation unit 461 observes people around the medical cart 15 and detects a patient demand for the medical cart 15 or perform generation and updating of a health state map.

The health state map is, for example, a map indicating the numbers of healthy people and unhealthy people or a ratio, a distribution, or the like thereof based on results obtained by checking healthy states of surrounding people while the medical cart 15 moves.

The medical control unit 462 controls a medical action by the medical cart 15 (the medical processing unit 422). For example, the medical control unit 462 performs control of telemedicine with the medical practitioner terminal 14. For example, the medical control unit 462 performs access control of medical information regarding each patient of the patient DB 12.

The movement control unit 463 controls movement of the medical cart 15 by controlling the driving unit 415 based on the patient demand or the like.

The output control unit 464 controls an output of various kinds of information by the output unit 418.

The communication control unit 465 controls communication processing by the communication unit 419.

### <Exemplary Configuration of Pharmacy Cart 16>

Fig. 8 illustrates an exemplary configuration of the pharmacy cart 16 of the telemedicine system 1 of Fig. 1 configured by the vehicle 301 of Fig. 5. In the drawing, the same reference numerals are given to the units corresponding to the medical cart 15 of Fig. 6 and description thereof will be appropriately omitted.

Compared to the medical cart 15, the pharmacy cart 16 similarly includes the processor 411, the ROM 412, the RAM 413, the recording medium 414, the driving unit 415, the input/output interface 416, the input unit 417, the output unit 418, the communication unit 419, the sensor group 420, the battery 421, and the bus 431 and differently includes a prescription processing unit 511 instead of the medical processing unit 422.

The prescription processing unit 511 is connected to the bus 431. The prescription processing unit 511 includes various prescription devices used for prescription and performs processing related to prescription. For example, the prescription processing unit 511 includes a prescription robot or the like that prescribes medicines remotely.

The prescription devices included in the prescription processing unit 511 may not all be devices that can be manipulated remotely or may be, for example, device that can be directly manipulated by pharmacists, patients, or the like.

The types of prescription devices included in the prescription processing unit 511 may be different from those of the pharmacy cart 16.

Hereinafter, description of the input/output interface 416 and the bus 431 will be omitted in description of the medical cart 15 and the pharmacy cart 16. For example, when the processor 411 and the output unit 418 transmit and receive data via the bus 431 and the input/output interface 416, mention of the bus 431 and the input/output interface 416 will be omitted. When the processor 411 and the output unit 418 mutually transmit and receive data, description thereof will be made.

### <Exemplary Configuration of Information Processing Unit 551>

Fig. 9 illustrates an exemplary configuration of the information processing unit 551 which is a part of a function realized by causing the processor 411 of the pharmacy cart 16 to execute a predetermined control program.

The information processing unit 551 includes an observation unit 561, a prescription control unit 562, a movement control unit 563, an output control unit 564, and a communication control unit 565.

The observation unit 561 observes the inside and the surroundings of the pharmacy cart 16 based on sensor data supplied from the sensor group 420. For example, the observation unit 561 observes a patient inside the pharmacy cart 16. For example, the observation unit 561 observes people around the pharmacy cart 16 and detects a patient demand for the pharmacy cart 16.

The prescription control unit 562 controls a prescription action by the pharmacy cart 16 (the prescription processing unit 511). For example, the prescription control unit 562 performs control of tele-prescription with the medical practitioner terminal 14. For example, the prescription control unit 562 performs access control of medical information regarding each patient of the patient DB 12.

The movement control unit 563 controls movement of the pharmacy cart 16 by controlling the driving unit 415.

The output control unit 564 controls an output of various kinds of information by the output unit 418.

The communication control unit 565 controls communication processing by the communication unit 419.

### <Processing of Telemedicine System 1>

Next, processing of the telemedicine system 1 will be described with reference to Figs. 10 to 22.

### <Processing of Server 11>

First, processing performed by the server 11 will be described with reference to the flowchart of Fig. 10.

For example, this processing starts when the server 11 is powered on, and ends when the server 11 is powered off.

In step S1, the communication unit 118 starts communication. That is, the communication unit 118 is connected to the network 31 under the control of the communication control unit 165 and starts communication with another device of the telemedicine system 1 as necessary.

In step S2, the organization unit 164 determines whether an organization of the medical cart 15 and the pharmacy cart 16 is updated. When it is determined that the organization of the medical cart 15 and the pharmacy cart 16 is updated, the processing proceeds to step S3.

Any timing at which the organization of the medical cart 15 and the pharmacy cart 16 is updated can be set. For example, the organization is updated periodically (for example, once per day). For example, the organization is updated when an event occurs in which a patient demand is considerably changed occurs (for example, when an epidemic of a disease (for example, influenza or the like), unseasonal weather, or disaster (for example, a typhoon, an earthquake, a fire, or the like)). For example, when states of the medical cart 15 and the pharmacy cart 16 are changed (for example, when the number of medical carts 15 and pharmacy carts 16 in operation is changed, when the telemedicine of a certain medical cart 15 ends, or when tele-prescription of a certain pharmacy cart 16 ends), the organization is updated.

In step S3, the server 11 performs organization processing. Thereafter, the processing proceeds to step S4.

Here, details of the organization processing will be described with reference to the flowchart of Fig. 11.

In step S51, the demand prediction unit 163 performs demand prediction.

Specifically, the demand prediction unit 163 collects various kinds of information used to predict a patient demand for the medical cart 15 and the pharmacy cart 16 from each medical practitioner terminal 14, each medical cart 15, each pharmacy cart 16, each patient terminal 17, another server (not illustrated), and the like via the network 31 and the communication unit 118.

For example, the demand prediction unit 163 collects a healthy state map from each medical cart 15. For example, the demand prediction unit 163 collects information regarding events (for example, prevalence of disease, weather, disaster, and the like) which cause a change in the demand of the patient. For example, the demand prediction unit 163 collects information indicating current positions or states of each medical cart 15 and each pharmacy cart 16.

As states of each medical cart 15 and each pharmacy cart 16, for example, states in which the telemedicine or tele-prescription is being executed, patrolling is being performed, waiting is being performed, and movement to a patient is being performed are assumed.

The demand prediction unit 163 may predict that an area where many people gave gathered or an area where there are many people in poor physical condition is, for example, an area where a demand for the medical cart 15 and the pharmacy cart 16 is high. For example, office streets on weekdays in daytime, station squares in the nighttime, residential areas on weekends, schools in which classes are closed, districts in which there are many aged people, and the like are predicted to be areas where the demand for the medical cart 15 and the pharmacy cart 16 will be high. For example, the demand prediction unit 163 may predict that an area where a disaster has occurred, an area in which disease is prevalent, an area with unseasonal weather or where seasonal fluctuations have occurred, or the like is an area where the demand for the medical cart 15 and the pharmacy cart 16 will be high.

In step S52, the organization unit 164 organizes the medical cart 15 and the pharmacy cart 16. For example, the organization unit 164 may set a location or a patrol route of each medical cart 15 and each pharmacy cart 16 based on the current positions and states of each medical cart 15 and each pharmacy cart 16 and the demand prediction. That is, the organization unit 164 sets the disposition or the patrol route of each medical cart 15 and each pharmacy cart 16 so that a larger number of medical carts 15 and pharmacy carts 16 gather in an area in which a patient demand will be higher.

In step S53, the organization unit 164 gives an instruction of disposition places or a patrol route to each medical cart 15 and each pharmacy cart 16. Specifically, the organization unit 164 generates information including the disposition places of each medical cart 15 and each pharmacy cart 16 (hereinafter referred to as a dispatch instruction information) or information including the patrol route (hereinafter referred to patrol route instruction information).

For example, the dispatch instruction information may include not only the disposition place but also, for example, a route to the disposition place and a time at which the disposition is started.

For example, the patrol route instruction information may include not only the patrol route but also, for example, a time when the patrolling according to the patrol route is performed and a target arrival time of each spot on the route.

The organization unit 164 transmits the dispatch instruction information and the patrol route instruction information to each medical cart 15 and each pharmacy cart 16 via the communication unit 118 and the network 31.

On the other hand, each medical cart 15 starts the patrolling according to the patrol route in step S205 of Fig. 14 or starts movement to the disposition place in step S207, as will be described below.

Each pharmacy cart 16 starts the patrolling according to the patrol route in step S403 of Fig. 19 or starts movement to the disposition place in step S405, as will be described below.

Thereafter, the organization processing ends.

In this processing, the disposition places or the patrol routes of all the medical carts 15 and the pharmacy carts 16 may not necessarily be set. For example, only the disposition places or the patrol routes of the medical carts 15 and the pharmacy carts 16 of which setting or changing of the disposition places or the patrol routes is necessary may be set.

Referring back to Fig. 10, conversely, when it is determined in step S2 that the organization of the medical cart 15 and the pharmacy cart 16 is not updated, the processing of step S3 is skipped and the processing proceeds to step S4.

In step S4, the organization unit 164 determines whether dispatching of the medical cart 15 is requested. For example, when information for requesting the dispatching of the medical cart 15 (hereinafter referred to as medical cart dispatching request information) transmitted by a patient or the like (for example, including an agent of the patient) using the patient terminal 17 is received via the network 31 and the communication unit 118, the organization unit 164 determines that the dispatching of the medical cart 15 is requested, and the processing proceeds to step S5.

The medical cart dispatching request information includes, for example, personal information of the patient (for example, a name, an age, a gender, a nationality, an address, a contact address, and an insurance card number, etc.), a patient state (for example, a condition, a symptom, or an injury state), a dispatch place, and a desired time. For example, biological information (for example, a body temperature, a pulse, or a blood pressure) of the patient measured by the patient terminal 17 (for example, a smartphone) may be included as a state of the patient in the medical cart dispatching request information.

For example, the patient may make a request for dispatching the medical cart 15 by a voice call.

In step S5, the server 11 dispatch the medical cart 15.

Specifically, the organization unit 164 selects the medical cart 15 to be dispatched. For example, the organization unit 164 selects the medical cart 15 which can supply telemedicine appropriate for the patient at a desired time near the dispatch place. The organization unit 164 generates information for giving an instruction for dispatching to the dispatch place (hereinafter referred to as medical cart dispatching instruction information). The communication unit 118 transmits the medical cart dispatching instruction information to the selected medical cart 15 via the network 31 under the control of the communication control unit 165. In this way, a dispatch destination of the medical cart 15 is designated based on the demand for patients.

The medical cart dispatching instruction information includes, for example, personal information of a patient, a state of the patient, a dispatch place, and an appointment time. The appointment time may not necessarily be the same as a desired time of the patient or may be set to a time different from the desired time in accordance with, for example, an activation situation or the like of each medical cart 15. For example, a route to the dispatch place may be included in the medical cart dispatching instruction information.

On the other hand, the medical cart 15 instructed to be dispatched starts moving to the dispatch place in step S207 of Fig. 14 to be described below.

Thereafter, the processing proceeds to step S6.

Conversely, when it is determined in step S4 that the dispatching of the medical cart 15 is not requested, the processing of step S5 is skipped and the processing proceeds to step S6.

In step S6, the organization unit 164 determines whether dispatching of the pharmacy cart 16 is requested. For example, when information for requesting the dispatching of the pharmacy cart 16 (hereinafter referred to as pharmacy cart dispatching request information) transmitted by a patient or the like (for example, including an agent of the patient) using the patient terminal 17 is received via the network 31 and the communication unit 118, the organization unit 164 determines that the dispatching of the pharmacy cart 16 is requested, and the processing proceeds to step S7.

The medical cart dispatching request information includes, for example, personal information of the patient (for example, a name, an age, a gender, a nationality, an address, a contact address, and an insurance card number, etc.), prescription data, a dispatch place, and a desired time.

For example, the patient may make a request for dispatching the pharmacy cart 16 by a voice call.

In step S7, the server 11 dispatch the pharmacy cart 16.

Specifically, the organization unit 164 selects the pharmacy cart 16 to be dispatched. For example, the organization unit 164 selects the pharmacy cart 16 which can dispense medicine supplied to the patient at a desired time near the dispatch place. The organization unit 164 generates information for giving an instruction for dispatching to the dispatch place (hereinafter referred to as pharmacy cart dispatching instruction information). The communication unit 118 transmits the pharmacy cart dispatching instruction information to the selected pharmacy cart 16 via the network 31 under the control of the communication control unit 165. In this way, a dispatch destination of the pharmacy cart 16 is designated based on the demand for patients.

The pharmacy cart dispatching instruction information includes, for example, personal information of a patient, prescription data, a dispatch place, and an appointment time. The appointment time may not necessarily be the same as a desired time of the patient or may be set to a time different from the desired time in accordance with, for example, an activation situation or the like of each pharmacy cart 16. For example, a route to the dispatch place may be included in the pharmacy cart dispatching instruction information.

On the other hand, the pharmacy cart 16 instructed to be dispatched starts moving to the disposition place in step S405 of Fig. 19 to be described below.

Thereafter, the processing proceeds to step S8.

Conversely, when it is determined in step S6 that the dispatching of the pharmacy cart 16 is not requested, the processing of step S7 is skipped and the processing proceeds to step S8.

In step S8, the selection unit 162 determines whether supply of the telemedicine is requested.

For example, when the patient using the medical cart 15 makes a request for supplying the telemedicine, this medical cart 15 (hereinafter referred to as an attention medical cart 15) transmits telemedicine request information for requesting supply of the telemedicine in step S251 of Fig. 16 to be described below.

When the telemedicine request information is received via the network 31 and the communication unit 118, the selection unit 162 determines that the supply of the telemedicine is requested and the processing proceeds to step S9.

The telemedicine request information includes, for example, personal information of the patient (for example, a name, an age, a gender, a nationality, an address, a contact address, and an insurance card number, etc.), a patient state (for example, a condition, a symptom, or an injury state), wishes of the patient, and content of a medical facility of the attention medical cart 15. The wishes of the patient include a desired condition of a medical practitioner executing the telemedicine and desired content of the telemedicine. The desired condition of the medical practitioner includes, for example, an age, a gender, working years, a specialized field (for example, a medical department or the like), a nationality, and usable languages of the medical practitioner. The desired content of the telemedicine includes, for example, an examination method, a curing method, and a budget.

In step S9, the server 11 performs medical practitioner matching processing. Thereafter, the processing proceeds to step S10.

Here, details of the medical practitioner matching processing will be described with reference to the flowchart of Fig. 12.

In step S101, the server 11 acquires medical information of the patient.

Specifically, in step S252 of Fig. 16 to be described below, the attention medical cart 15 performs processing for permitting access to the medical information of the patient receiving the telemedicine in the patient DB 12.

On the other hand, the analysis unit 161 reads the medical information of the patient from the patient DB 12 via the communication unit 118 and the network 31.

In step S102, the analysis unit 161 analyzes the state of the patient. For example, the analysis unit 161 analyzes the condition of the patient, the symptom, the injury state, or the like based on a state reported from the patient, the acquired medical information, and the like. The analysis unit 161 selects a telemedicine action necessary for the patient based on an analysis result.

In step S103, the selection unit 162 searches for medical practitioners. Specifically, the selection unit 162 accesses the medical DB 13 via the communication unit 118 and the network 31 and searches for medical practitioners who are available at a current time point and can execute telemedicine actions necessary for the patient. When the patient requests an introduction of other medical practitioners in step S106 to be described below, the medical practitioners introduced so far are excluded from searching targets. Further, the selection unit 162 calculates a matching ratio with the desired condition of the patient of each of the searched medical practitioners and determines a medical practitioner with a matching ratio equal to or greater than a predetermined threshold as medical practitioners who can be introduced to the patient.

In step S104, the analysis unit 161 determines whether the supply of the telemedicine is possible. For example, when the telemedicine action necessary for the patient can be executed using the medical device of the attention medical cart 15, the telemedicine action is not against the wishes of the patient, and the medical practitioners who can be introduced to the patient are searched for, the analysis unit 161 determines that the supply of the telemedicine is possible and the processing proceeds to step S105.

In step S105, the selection unit 162 introduces the medical practitioners. For example, the selection unit 162 selects a predetermined number of medical practitioners in order in which the matching ratio with the desired condition of the patient is higher among the medical practitioners who are searched for in the processing of step S103 and can be introduced to the patient as the medical practitioners introduced to the patient. The selection unit 162 generates medical practitioner introduction information including information regarding the selected medical practitioners. The communication unit 118 transmits the medical practitioner introduction information to the attention medical cart 15 via the network 31 under the control of the communication control unit 165.

The medical practitioner introduction information includes, for example, information regarding a facial photo, a profile, a field of specialization, a career, or the like of each medical practitioner or a uniform resource locator (URL) of a website from which information regarding each medical practitioner can be acquired.

Any number of medical practitioners to be introduced can be used and may be set by, for example, a patient.

In step S106, the selection unit 162 determines whether introduction of other medical practitioners is requested.

For example, when the patient makes a request for introducing the other medical practitioners, the attention medical cart 15 transmits medical practitioner reintroduction request information for requesting the introduction of the other medical practitioners in step S259 of Fig. 16 to be described below.

When the medical practitioner introduction request information is received via the network 31 and the communication unit 118, the selection unit 162 determines that the introduction of the other medical practitioners is requested and the processing returns to step S102.

The medical practitioner introduction request information includes, for example, personal information regarding the patient, a state of the patient, and a wish of the patient. Such information indicates a state in which transmission from the attention medical cart 15 to the server 11 is already finished in accordance with the telemedicine request information and is appropriately changed by the patient or the like, for example, to deliver the state or the wish of the patient in more detail or change a searching condition of the medical practitioners.

Thereafter, the processing of steps S102 to S106 is repeatedly performed until it is determined in step S104 the supply of the telemedicine is not possible or it is determined in step S106, the introduction of the other medical practitioners is not requested.

Conversely, when it is determined in step S106 that the introduction of the other medical practitioners is not requested, for example, when the patient selects the introduced medical practitioners or does not execute an examination of the telemedicine, the selection unit 162 ends the medical practitioner matching processing.

In step S104, for example, when the telemedicine action necessary for the patient cannot be executed using the medical devices of the attention medical cart 15, the telemedicine action necessary for the patient is against the wish of the patient, or the medical practitioners who can be introduced to the patient are not searched for, the analysis unit 161 determines that the supply of the telemedicine is not possible, and the processing proceeds to step S107.

In step S107, the server 11 proposes an alternative.

For example, the analysis unit 161 generates alternative information indicating the alternative in accordance with a reason why the telemedicine cannot be supplied. For example, when the telemedicine action necessary for the patient cannot be executed using the medical devices of the attention medical cart 15, the analysis unit 161 generates the alternative information for proposing close medical facilities capable of executing the medical action. For example, when the telemedicine action necessary for the patient is against the wish of the patient, the analysis unit 161 generates the alternative information for proposing the telemedicine action against the wish of the patient. For example, when the medical practitioners who can be introduced to the patient are not searched for, the analysis unit 161 generates the alternative information for proposing a period of time in which the medical practitioners who can be introduced to the patient are available or a close medical organization in which there are the medical practitioners who can be introduced to the patient.

The communication unit 118 transmits the alternative information to the attention medical cart 15 via the network 31 under the control of the communication control unit 165.

Thereafter, the medical practitioner matching processing ends.

Referring back to Fig. 10, conversely, when it is determined in step S8 that the supply of the telemedicine is not requested, the processing of step S9 is skipped and the processing proceeds to step S10.

In step S10, the selection unit 162 determines whether supply of the tele-prescription is requested.

For example, when the patient using the pharmacy cart 16 makes a request for supplying the tele-prescription, the pharmacy cart 16 (hereinafter referred to as an attention pharmacy cart 16) transmits tele-prescription request information indicating a request for supplying the tele-prescription in step S451 of Fig. 20 to be described below.

When the tele-prescription request information is received via the network 31 and the communication unit 118, the selection unit 162 determines that the supply of the tele-prescription is requested and the processing proceeds to step S11.

The tele-prescription request information includes, for example, personal information of the patient (for example, a name, an age, a gender, a nationality, an address, a contact address, and an insurance card number, etc.), a patient state (for example, a condition, a symptom, or an injury state), wishes of the patient, prescription data, and content of a prescription facility of the attention pharmacy cart 16. The wishes of the patient include a desired condition of a pharmacist executing the tele-prescription and desired content of the tele-prescription. The desired condition of the pharmacist includes, for example, an age, a gender, working years, a nationality, and usable languages of the pharmacist. The desired content of the tele-prescription includes, for example presence or absence of generic drugs and a budget.

In step S11, the server 11 performs pharmacist matching processing. Thereafter, the processing returns to step S2 and proceeds to processing subsequent to step S2.

Here, details of the pharmacist matching processing will be described with reference to the flowchart of Fig. 13.

In step S151, the server 11 acquires medical information of the patient.

Specifically, in step S452 of Fig. 20 to be described below, the attention pharmacy cart 16 performs processing for permitting access to the medical information of the patient receiving the tele-prescription in the patient DB 12.

On the other hand, the analysis unit 161 reads the medical information of the patient from the patient DB 12 via the communication unit 118 and the network 31.

The medical information of the patient includes, for example, a medication history, an allergy information, and the like of the patient. For example, access to a clinical card of the patient is prohibited because the clinical card is not necessary for the prescription.

In step S152, the selection unit 162 searches for pharmacists. Specifically, the selection unit 162 accesses the medical DB 13 via the communication unit 118 and the network 31 and searches for pharmacists who are available at a current time point. When the patient requests an instruction of other pharmacists in step S155 to be described below, the pharmacists introduced so far are excluded from searching targets. Further, the selection unit 162 calculates a matching ratio with the desired condition of the patient of each of the searched pharmacists and determines a pharmacist with a matching ratio equal to or greater than a predetermined threshold as pharmacists who can be introduced to the patient.

In step S153, the analysis unit 161 determines whether the supply of the tele-prescription is possible. For example, when the medicine based on the prescription data can be dispensed using the prescription processing unit 511 of the attention pharmacy cart 16 and the pharmacists who can be introduced to the patient are searched for, the analysis unit 161 determines that the supply of the tele-prescription is possible and the processing proceeds to step S154.

In step S154, the selection unit 162 introduces the pharmacists. For example, the selection unit 162 selects a predetermined number of pharmacists in order in which the matching ratio with the desired condition of the patient is higher among the pharmacists who are searched for in the processing of step S152 and can be introduced to the patient as the pharmacists introduced to the patient. The selection unit 162 generates pharmacist introduction information including information regarding the selected pharmacists. The communication unit 118 transmits the pharmacist introduction information to the attention pharmacy cart 16 via the network 31 under the control of the communication control unit 165.

The pharmacist introduction information includes, for example, information regarding a facial photo, a profile, a field of specialization, a career of each pharmacist or a URL of a website from which information regarding each pharmacist can be acquired.

Any number of pharmacists to be introduced can be used and may be set by, for example, a patient.

In step S155, the selection unit 162 determines whether introduction of other pharmacists is requested.

For example, when the patient makes a request for introducing the other pharmacists, the attention pharmacy cart 16 transmits pharmacist introduction request information for requesting the introduction of the other pharmacists in step S459 of Fig. 20 to be described below.

When the pharmacist introduction request information is received via the network 31 and the communication unit 118, the selection unit 162 determines that the introduction of the other pharmacists is requested and the processing returns to step S152.

The pharmacist introduction request information includes, for example, personal information regarding the patient, a state of the patient, and a wish of the patient. Such information indicates a state in which transmission from the attention pharmacy cart 16 to the server 11 is already finished in accordance with the tele-prescription request information and is appropriately changed by the patient or the like, for example, to deliver the state or the wish of the patient in more detail or change a searching condition of the pharmacists.

Thereafter, the processing of steps S152 to S155 is repeatedly performed until it is determined in step S153 that the supply of the tele-prescription is not possible or it is determined in step S155 that the introduction of the other pharmacists is not requested.

Conversely, when it is determined in step S155 that the introduction of the other pharmacists is not requested, for example, when the patient selects the introduced pharmacists or does not execute an examination of the tele-prescription, the selection unit 162 ends the pharmacist matching processing.

For example, when the medicine based on the prescription data cannot be dispensed using the prescription processing unit 511 of the attention pharmacy cart 16 and the pharmacists who can be introduced to the patient are not searched for in step S153, the analysis unit 161 determines that the supply of the tele-prescription is not possible and the processing proceeds to step S156.

In step S156, the server 11 proposes an alternative.

For example, the analysis unit 161 generates alternative information indicating the alternative in accordance with a reason why the telemedicine is not supplied. For example, when the medicine based on the prescription data cannot be dispensed using the prescription processing unit 511 of the attention pharmacy cart 16, the analysis unit 161 generates the alternative information for proposing close pharmacies capable of executing the prescription. For example, when the pharmacists who can be introduced to the patient are not searched for, the analysis unit 161 generates the alternative information for proposing a period of time in which the pharmacists who can be introduced to the patient are available or a close pharmacy in which there are the pharmacists who can be introduced to the patient.

The communication unit 118 transmits the alternative information to the attention pharmacy cart 16 via the network 31 under the control of the communication control unit 165.

Thereafter, the pharmacist matching processing ends.

Referring back to Fig. 10, conversely, when it is determined in step S10 that the supply of the tele-prescription is not requested, the processing returns to step S2 and the processing subsequent to step S2 is performed.

### <Processing of Medical Cart 15>

Next, processing performed by the medical cart 15 to correspond to the processing of the server 11 of Fig. 10 will be described with reference to the flowcharts of Figs. 14 and 15.

For example, this processing starts when a power source for electric components of the medical cart 15 is turned on, and ends when the power source is turned off.

In step S201, the communication unit 419 starts communication. That is, the communication unit 419 is connected to the network 31 under the control of the communication control unit 465 to start communication with other devices of the telemedicine system 1 as necessary.

In step S202, the medical cart 15 starts observing surroundings.

For example, the cameras 312F to 312B of the medical cart 15 starts processing for photographing the surroundings of the medical cart 15 and supplying obtained surrounding images to the processor 411 or storing the obtained surrounding images in the RAM 413 or the recording medium 414.

The observation unit 461 starts observing surroundings of the medical cart 15 based on the surrounding images. For example, the observation unit 461 starts processing for estimating healthy states of surrounding people based on complexions, expressions, motions, or the like of people around the medical cart 15.

In step S203, the observation unit 461 starts updating a healthy state map. Specifically, the observation unit 461 starts updating the healthy state map indicating the numbers of healthy people and unhealthy people or a ratio, a distribution, or the like thereof based on an estimation result of the healthy states of people around the medical cart 15. The communication unit 419 starts processing for transmitting the healthy state map to the server 11 via the network 31 under the control of the communication control unit 465 as necessary.

In step S204, the movement control unit 463 determines whether an instruction of a patrol route is given. When the patrol route instruction information transmitted by the server 11 in the processing of step S53 of Fig. 11, as described above, is received via the network 31 and the communication unit 419, the movement control unit 463 determines that the instruction of the patrol route is given, and the processing proceeds to step S205.

In step S205, the medical cart 15 starts patrolling. Specifically, the movement control unit 463 starts processing for controlling the driving unit 415 so that the patrol route indicated by the patrol route instruction information is toured. When a target arrival time of each transit point on the patrol route is set, the movement control unit 463 starts processing for controlling the driving unit 415 so that the medical cart 15 arrives at each transit point at the set target arrival time.

Thereafter, the processing proceeds to step S206.

Conversely, when it is determined in step S204 that the instruction of the patrol route is not given, the processing of step S205 is skipped and the processing proceeds to step S206.

In step S206, the movement control unit 463 determines whether the destination is the instructed destination. When the medical cart dispatching instruction information transmitted by the server 11 in the processing of step S5 of Fig. 10, as described above, or the dispatch instruction information transmitted by the server 11 in the processing of step S53 of Fig. 11, as described above, is received via the network 31 and the communication unit 419, the movement control unit 463 determines that the instruction of the destination is given and the processing proceeds to step S207.

In step S207, the medical cart 15 starts movement to the destination. Specifically, the movement control unit 463 sets a dispatch place indicated by the medical cart dispatching instruction information or a dispatch place indicated by the dispatch instruction information as the destination. Then, the movement control unit 463 starts processing for controlling the driving unit 415 so that the medical cart 15 is moved to the destination. When an instruction of a route to the destination is given, the movement control unit 463 starts processing for controlling the driving unit 415 so that the medical cart 15 is moved along the instructed route. Further, when a target arrival time of each transit point on the route is set, the movement control unit 463 starts processing for controlling the driving unit 415 so that the medical cart 15 arrives at each transit point at the set target arrival time. When an appointment time is set, the movement control unit 463 starts processing for controlling the driving unit 415 so that the medical cart 15 arrives at the destination in the set appointment time.

Thereafter, the processing proceeds to step S208.

Conversely, when neither the medical cart dispatching instruction information nor the dispatch instruction information is received in step S206, the movement control unit 463 determines that the instruction of the destination is not given, the processing of step S207 is skipped, and the processing proceeds to step S208.

In step S208, the movement control unit 463 determines whether the medical cart 15 arrives at the destination. Specifically, when the destination is set, the movement control unit 463 detects a current position of the medical cart 15 based on the sensor data from a positional sensor included in the sensor group 268. When the current position of the medical cart 15 matches the destination, the movement control unit 463 determines that the medical cart 15 arrives at the destination and the processing proceeds to step S209.

Here, the destination is a dispatch place, a dispatch place, or a transportation place to be described below.

In step S209, the medical cart 15 is stopped under the control of the movement control unit 463.

Thereafter, the processing proceeds to step S210.

Conversely, when the current position of the medical cart 15 does not match the destination or the destination is not set in step S208, the movement control unit 463 determines that the medical cart 15 does not arrives at the destination, and the processing of step S209 is skipped, and the processing proceeds to step S210.

In step S210, the observation unit 461 determines whether there is a person for whom the telemedicine is necessary. When the observation unit 461 determines that there is a person for whom the telemedicine is necessary around the medical cart 15 as a result obtained by observing the surroundings of the medical cart 15, the processing proceeds to step S211.

Here, a person for whom the telemedicine is necessary is, for example, a person who is estimated to have poor healthy states apparently and receive certain medical actions quickly.

In step S211, the movement control unit 463 determines whether a patient is not being transported. When it is determined that the patient is not being transported, the processing proceeds to step S212.

In step S212, the medical cart 15 is stopped as in the processing of step S209. Thus, the medical cart 15 is stopped near a person who is a target on which the telemedicine is executed, that is, a person for whom it is determined that the telemedicine is necessary.

For example, the output unit 418 guides the person for whom it is determined that the telemedicine is necessary to board the medical cart 15 by an image, a sound, or the like under the control of the output control unit 464. When the person for whom it is determined that the telemedicine is necessary board the stopped medical cart 15, the person can receive the telemedicine.

Thereafter, the processing proceeds to step S213.

Conversely, when it is determined in step S211 that the patient is being transported, the processing of step S212 is skipped and the processing proceeds to step S213. That is, when the patient is being transported to a close medical organization, home, or the like in the processing of step S267 to be described below, the transportation of the patient is preferred.

When it is determined in step S210 that there is no person for whom the telemedicine is necessary, the processing of steps S211 and S212 is skipped and the processing proceeds to step S213.

In step S213, the observation unit 461 determines whether a surrounding person calls and halts the medical cart 15. For example, when a person making a gesture to stop the medical cart 15 is detected as a result obtained by observing the surroundings of the medical cart 15, the observation unit 461 determines that the surrounding person calls and halts the medical cart 15 and the processing proceeds to step S214.

In step S214, the movement control unit 463 determines whether an appointment is not made. When it is determined that the appointment is not made, the processing proceeds to step S215.

In step S215, it is determined whether the patient is being transported as in the processing of step S211. When it is determined that the patient is not being transported, the processing proceeds to step S216.

In step S216, the medical cart 15 is stopped as in the processing of step S209. Thus, the medical cart 15 is stopped near a person who is a telemedicine execution target, that is, a person calling and halting the medical cart 15, or his or her companion. Then, when the person calling and halting the medical cart 15 or his or her companion boards the stopped medical cart 15, the person or his or companion can receive the telemedicine.

Thereafter, the processing proceeds to step S217.

Conversely, when it is determined in step S215 that the patient is being transported, the processing of step S216 is skipped and the processing proceeds to step S217. That is, the transportation of the patient is preferred and the medical cart 15 does not response to the calling and halting of a passenger.

When it is determined in step S214 that tan appointment is made, the processing of steps S215 and S216 is skipped and the processing proceed to step S217. For example, when the dispatch of the medical cart 15 is already requested, it is determined that the appointment is made and the processing proceeds to step S217. That is, the medical cart 15 prefers the appointment and does not respond to the calling and halting of a surrounding person.

Further, when it is determined in step S213 that the surrounding person does not call and halt, the processing of steps S214 to S216 is skipped and the processing proceeds to step S217.

In step S217, the medical control unit 462 determines whether the telemedicine is executed. For example, when the patient performs an operation of executing an examination for the telemedicine via the input unit 417, the medical control unit 462 determines to execute the telemedicine and the processing proceeds to step S218.

In step S218, the medical cart 15 executes telemedicine execution processing. Thereafter, the processing returns to step S204 and processing subsequent to step S204 is performed.

Here, details of the telemedicine execution processing will be described with reference to the flowcharts of Figs. 16 and 17.

In step S251, the medical control unit 462 makes a request for supplying the telemedicine. Specifically, the medical control unit 462 generates the above-described telemedicine request information. The communication unit 419 transmits the telemedicine request information to the server 11 via the network 31 under the control of the communication control unit 465.

In step S252, the medical control unit 462 permits access to the medical information of the patient of the patient DB 12.

A method of permitting access to the medical information of the patient of the patient DB 12 is not particularly limited. For example, the medical control unit 462 issues a one-time password for accessing the medical information of the patient. The communication unit 419 transmits the one-time password to the server 11 via the network 31 under the control of the communication control unit 465. The server 11 can access the medical information of the patient of the patient DB 12 using the received one-time password.

In step S253, the medical control unit 462 determines whether the medical practitioner is introduced. When it is determined that the medical practitioner is not instructed, the processing proceeds to step S254.

In step S254, the medical control unit 462 determines whether an alternative is proposed. When it is determined that the alternative is not proposed, the processing returns to step S253.

Thereafter, until it is determined in step S253 that the medical practitioner is introduced or it is determined in step S254 that the alternative is proposed, the processing of steps S253 and S254 is repeatedly performed.

Conversely, when the alternative information transmitted by the server 11 in the processing of step S107 of Fig. 12, as described above, is received via the network 31 and the communication unit 419 in step S254, the medical control unit 462 determines that the alternative is proposed and the processing proceeds to step S255.

In step S255, the medical cart 15 proposes the alternative. Specifically, the output unit 418 outputs at least one of visual information and auditory information for notifying the alternative indicated by the alternative information under the control of the output control unit 464. For example, a screen on which the alternative is proposed is displayed on the output unit 418 or a sound proposing the alternative is output from the output unit 418.

Thereafter, the processing proceeds to step S260.

Conversely, when the medical practitioner introduction information transmitted from the server 11 in step S105 of Fig. 12, as described above, is received via the network 31 and the communication unit 419 in step S253, the medical control unit 462 determines that the medical practitioner is introduced and the processing proceeds to step S256.

In step S256, the medical cart 15 proposes information regarding the introduced medical practitioner. For example, the output unit 418 displays a screen on which the information regarding the medical practitioner indicated in the medical practitioner introduction information is shown under the control of the output control unit 464.

In step S257, the medical control unit 462 determines whether the introduced medical practitioner is selected. When it is determined that the introduced medical practitioner is not selected, the processing proceeds to step S258.

In step S258, the medical control unit 462 determines whether the introduction of another medical practitioner is requested. For example, when the patient performs an operation of requesting the introduction of the other medical practitioner via the input unit 417, the medical control unit 462 determines that the introduction of the other medical practitioner is requested and the processing proceeds to step S259.

In step S259, the medical cart 15 makes a request for introducing the other medical practitioner. Specifically, the medical control unit 462 generates the above-described medical practitioner introduction request information. The communication unit 419 transmits the medical practitioner introduction request information to the server 11 via the network 31 under the control of the communication control unit 465.

Thereafter, the processing returns to step S253 and the processing subsequent to step S253 is performed.

Conversely, when the patient does not perform the operation of requesting the introduction of the other medical practitioner in step S258, the medical control unit 462 determines that the introduction of the other medical practitioner is not requested and the processing proceeds to step S260. This is a case in which the introduced medical practitioner is not selected and the introduction of the other medical practitioner is not requested.

In step S260, the medical control unit 462 prohibits access to the medical information of the patient of the patient DB 12.

Thereafter, the telemedicine execution processing ends.

Conversely, for example, when the patient performs the operation of selecting the introduced medical practitioner via the input unit 417 in step S257, the medical control unit 462 determines that the introduced medical practitioner is selected and the processing proceeds to step S261.

Thus, for example, when one medical practitioner is introduced and the introduced medical practitioner is selected and two or more medical practitioners are introduced, and a desired medical practitioner is selected from them.

In step S261, the medical cart 15 starts communication with the selected medical practitioner. Specifically, the communication unit 419 starts communication with the medical practitioner terminal 14 of the selected medical practitioner via the network 31 under the control of the communication control unit 465.

For example, the communication unit 419 starts transmitting an image and a sound of the patient to the medical practitioner terminal 14 under the control of the communication control unit 465 and starts receiving an image and a sound of the medical practitioner from the medical practitioner terminal 14. Thus, the patient and the medical practitioner start telecommunication using the mutual images and sounds.

In step S262, the medical control unit 462 determines whether telemanipulation data of the medical device is received.

For example, when the medical practitioner performs a telemanipulation on the medical device included in the medical processing unit 422 of the medical cart 15, the medical practitioner inputs an instruction of the telemanipulation to the medical practitioner terminal 14. The medical practitioner terminal 14 generates telemanipulation data indicating manipulation content in the telemanipulation in step S303 of Fig. 18 to be described below and transmits the telemanipulation data to the medical cart 15.

When the medical control unit 462 determines that the telemanipulation data is received from the medical practitioner terminal 14 via the network 31 and the communication unit 419, the processing proceeds to step S263.

In step S263, the medical control unit 462 controls an operation of the medical device included in the medical processing unit 422 based on the telemanipulation data. Thus, the telemedicine can be supplied to the patient using the medical device.

Thereafter, the processing proceeds to step S264.

Conversely, when it is determined in step S262 that the telemanipulation data of the medical device is not received, the processing of step S263 is skipped and the processing proceeds to step S264.

In step S264, the medical control unit 462 determines whether the prescription data is received.

For example, when the medical practitioner issues the prescription for the patient based on a result of the telemedicine, the medical practitioner input the prescription data to the medical practitioner terminal 14. The medical practitioner terminal 14 transmits the input prescription data to the medical cart 15 in step S305 of Fig. 18 to be described below.

When the medical control unit 462 determines that the prescription data is received from the medical practitioner terminal 14 via the network 31 and the communication unit 419, the processing proceeds to step S265.

In step S265, the medical cart 15 stores the prescription data. For example, the medical control unit 462 stores the prescription data in the recording medium 414. For example, the communication unit 419 transmits the prescription data to the patient terminal 17 of the patient via the network 31 under the control of the communication control unit 465 to store the prescription data.

Thereafter, the processing proceeds to step S266.

Conversely, when it is determined in step S264 that the prescription data is not received, the processing of step S265 is skipped and the processing proceeds to step S266.

In step S266, the movement control unit 463 determines whether an instruction to transport the patient is given.

For example, when the medical practitioner determines that it is better to transport the patient to a medical organization, home, or the like as a result obtained by executing the telemedicine of the patient, the medical practitioner inputs the instruction to transport the patient to the medical practitioner terminal 14. The medical practitioner terminal 14 generates transportation instruction information to give the instruction to transport the patient in step S307 of Fig. 18 to be described below and transmits the transportation instruction information to the medical cart 15.

When the transportation instruction information is received via the network 31 and the communication unit 419, the movement control unit 463 determines that the instruction to transport patient is received and the processing proceeds to step S267.

The transportation instruction information includes, for example, personal information of the patient (for example, a name, an age, a gender, a nationality, an address, a contact address, and an insurance card number, etc.), a patient state (for example, a condition, a symptom, or an injury state), a transportation place (for example, a medical organization, home, or the like), and identification information of the medical cart 15 used by the patient.

In step S267, the medical cart 15 starts transporting the patient.

For example, when a transportation destination of the transportation instruction information and medical organization is set, the movement control unit 463 accesses the medical DB 13 via the communication unit 419 and the network 31 and searches for medical organization which can accept the patient near the medical cart 15. The movement control unit 463 sets the searched medical organization as a destination.

For example, the medical practitioners may designate a specific medical organization as a transportation place.

For example, when a home of the patient is set as the transportation destination of the transportation instruction information, the movement control unit 463 sets the home of the patient as a destination.

The movement control unit 463 starts processing for controlling the driving unit 415 so that the medical cart 15 moves to the destination.

Thereafter, the processing proceeds to step S268.

Conversely, when it is determined in step S266 that the medical practitioner determines that the instruction to transport the patient is not given, the processing of step S267 is skipped and the processing proceeds to step S268.

In step S268, it is determined whether the medical cart arrives at the destination (in this case, the transportation place), as in the processing of step S208 of Fig. 14. When it is determined that the medical cart arrives at the destination, the processing proceeds to step S269.

In step S269, the medical cart 15 is stopped as in the processing of step S209 of Fig. 14.

Thereafter, the processing proceeds to step S270.

Conversely, when it is determined in step S268 that the medical cart does not arrive at the destination, the processing of step S269 is skipped and the processing proceeds to step S270.

In step S270, the medical control unit 462 determines whether the telemedicine ends. When it is determined that the telemedicine does not end, the processing returns to step S262.

Thereafter, until it is determined in step S270 that the telemedicine ends, the processing of steps S262 to S270 is repeatedly performed.

Thus, for example, the medical practitioner remotely executes an examination, treatments, medical consultation, health consultation, medicine prescription, counseling, therapy, or the like for the patient while remotely manipulating the medical device of the medical cart 15, as necessary, using the medical practitioner terminal 14. The patient is transported to a close medical organization, his or her home, or the like as necessary.

Conversely, for example, when the patient performs a manipulation to end the examination of the telemedicine via the input unit 417 in step S270, the medical control unit 462 determines that the telemedicine ends. The medical control unit 462 generates telemedical examination end information to notify of end of the telemedical examination. The communication unit 419 transmits the telemedical examination end information to the medical practitioner terminal 14 of the medical practitioner via the network 31 under the control of the communication control unit 465. Thereafter, the processing proceeds to step S271.

For example, when the medical practitioner inputs an instruction to end the supply of the telemedicine to the medical practitioner terminal 14 in step S308 of Fig. 18 to be described below, the medical practitioner terminal 14 generates telemedicine supply end information to notify of end of the supply of the telemedicine and transmits the telemedicine supply end information to the medical cart 15.

When the telemedicine supply end information is received from the medical practitioner terminal 14 via the network 31 and the communication unit 419 in step S270, the medical control unit 462 determines that the telemedicine ends and the processing proceeds to step S271.

In step S271, the medical cart 15 ends the communication with the medical practitioner. That is, the communication unit 419 disconnects the communication with the medical practitioner terminal 14 of the medical practitioner executing the telemedicine under the control of the communication control unit 465.

In step S272, the access to the medical information of the patient is prohibited as in the processing of step S260.

Thereafter, the telemedicine execution processing ends.

Referring back to Fig. 15, conversely, when it is determined in step S217 that the telemedicine is not executed, the processing returns to step S204 and the processing subsequent to step S204 is performed.

### <Telemedicine Control Processing>

Next, telemedicine control processing performed by the medical practitioner terminal 14 of the medical practitioner to correspond to the telemedicine execution processing of the medical cart 15 in Figs. 16 and 17 will be described with reference to the flowchart of Fig. 18.

In step S301, the medical practitioner terminal 14 starts communication with the patient. Specifically, the communication unit 218 starts communication with the medical cart 15 used by the patient via the network 31 to correspond to the processing of the medical cart 15 of step S261 of Fig. 17, as described above.

In step S302, the processor 211 determines whether the telemanipulation is performed on the medical device of the medical cart 15.

For example, when the medical practitioner performs a telemanipulation of the medical device included in the medical processing unit 422 of the medical cart 15, the medical practitioner inputs an instruction of the telemanipulation via the input unit 216. When an input of the instruction of the telemanipulation is detected, the processor 211 determines that the telemanipulation of the medical device of the medical cart 15 is performed and the processing proceeds to step S303.

In step S303, the medical practitioner terminal 14 transmits telemanipulation data of the medical device of the medical cart 15. Specifically, the processor 211 generates the above-described telemanipulation data and transmits the telemanipulation data to the medical cart 15 via the communication unit 218 and the network 31.

Thereafter, the processing proceeds to step S304.

Conversely, when it is determined in step S302 that the telemanipulation is not performed on the medical device of the medical cart 15, the processing of step S303 is skipped and the processing proceeds to step S304.

In step S304, the processor 211 determines whether prescription data is input.

For example, when the medical practitioner issues a prescription for the patient based on a result of the telemedicine, the medical practitioner inputs the prescription data to the medical practitioner terminal 14 via the input unit 216. When the processor 211 determines that the prescription data is input, the processing proceeds to step S305.

In step S305, the processor 211 transmits the prescription data to the medical cart 15 via the communication unit 218 and the network 31.

Thereafter, the processing proceeds to step S306.

Conversely, when it is determined in step S304 that the prescription data is not input, the processing of step S305 is skipped and the processing proceeds to step S306.

In step S306, the processor 211 determines whether an instruction to transport the patient is given.

For example, when the medical practitioner gives the instruction to transport the patient to the medical cart 15, the medical practitioner inputs the instruction to transport the patient via the input unit 216. When the input of the instruction to transport the patient is detected, the processor 211 determines that the instruction to transport the patient is given and the processing proceeds to step S307.

In step S307, the medical practitioner terminal 14 gives the instruction to transport the patient. Specifically, the processor 211 generates the above-described transportation instruction information and transmits the transportation instruction information to the medical cart 15 via the communication unit 218 and the network 31.

Thereafter, the processing proceeds to step S308.

Conversely, when it is determined in step S306 that the instruction to transport the patient is not given, the processing of step S307 is skipped and the processing proceeds to step S308.

In step S308, the processor 211 determines whether the telemedicine ends. When it is determined that the telemedicine does not end, the processing returns to step S302.

Thereafter, until it is determined in step S308 that the telemedicine ends, the processing of steps S302 to S308 is repeatedly performed.

Conversely, for example, when the medical practitioner performs a manipulation to end the supply of the telemedicine via the input unit 216 in step S308, the processor 211 determines that the telemedicine ends. The processor 211 generates the above-described telemedicine supply end information and transmits the telemedicine supply end information to the medical cart 15 of the patient via the communication unit 218 and the network 31. Thereafter, the processing proceeds to step S309.

For example, when the telemedical examination end information transmitted by the medical cart 15 in the processing of step S270 of Fig. 17, as described above, is received via the network 31 and the communication unit 218, the processor 211 determines that the telemedicine ends and the processing proceeds to step S309.

In step S309, the medical practitioner terminal 14 ends the communication with the patient. That is, the communication unit 218 disconnects the communication with the medical cart 15 of the patient supplying the telemedicine.

Thereafter, the telemedicine control processing ends.

### <Processing of Pharmacy Cart 16>

Next, processing performed by the pharmacy cart 16 to correspond to the processing of the server 11 of Fig. 10 will be described with reference to the flowchart of Fig. 19.

For example, this processing starts when a power source for electric components of the pharmacy cart 16 is turned on, and ends when the power source is turned off.

In steps S401 to S403, the same processing as that of steps S201 to S203 of the medical cart 15 in Fig. 14 is performed.

In step S404, the movement control unit 563 determines whether the destination is the instructed destination. When the pharmacy cart dispatching instruction information transmitted by the server 11 in the processing of step S7 of Fig. 10, as described above, or the dispatch instruction information transmitted by the server 11 in the processing of step S53 of Fig. 11, as described above, is received via the network 31 and the communication unit 419, the movement control unit 563 determines that the instruction of the destination is given and the processing proceeds to step S405.

In step S405, movement to the destination starts in the same processing as that of step S207 of the medical cart 15 in Fig. 14.

Thereafter, the processing proceeds to step S406.

Conversely, when neither the pharmacy cart dispatching instruction information nor the dispatch instruction information is received in step S404, the movement control unit 563 determines that the instruction of the destination is not given, the processing of step S405 is skipped, and the processing proceeds to step S406.

In step S406, it is determined whether to arrive at the destination in the same processing as that of step S208 of the medical cart 15 in Fig. 14. When it is determined that the pharmacy carts arrives at the destination, the processing proceeds to step S407.

Here, the destination is a dispatch place or a dispatch place.

In step S407, the pharmacy cart 16 is stopped under the control of the movement control unit 563.

Thereafter, the processing proceeds to step S408.

Conversely, when it is determined in step S406 that the pharmacy carts does not arrive at the destination, the processing of step S407 is skipped and the processing proceeds to step S408.

In step S408, it is determined whether a surrounding person calls and halts in the same processing as that of step S213 of the medical cart 15 in Fig. 15. When it is determined that the surrounding person calls and halts, the processing proceeds to step S409.

In step S409, the movement control unit 563 determines whether an appointment is made. When it is determined that the appointment is not made, the processing proceeds to step S410.

In step S410, the pharmacy cart 16 is stopped as in the processing of step S407. Thus, the pharmacy cart 16 is stopped near a person who is a tele-prescription execution target, that is, a person calling and halting the pharmacy cart 16, or his or her companion. Then, when the person calling and halting the pharmacy cart 16 or his or her companion boards the stopped pharmacy cart 16, the person or his or companion can receive the tele-prescription.

Thereafter, the processing proceeds to step S411.

Conversely, when it is determined in step S409 that the appointment is made, the processing of step S410 is skipped and the processing proceed to step S411. For example, when the dispatch of the pharmacy cart 16 is already requested, it is determined that the appointment is made and the processing proceeds to step S411. That is, the pharmacy cart 16 prefers the appointment and does not respond to the calling and halting of a surrounding person.

Further, when it is determined in step S408 that the surrounding person does not call and halt, the processing of steps S409 and S410 is skipped and the processing proceeds to step S411.

In step S411, the prescription control unit 562 determines whether the tele-prescription is executed. For example, when the patient performs an operation to receive the tele-prescription is performed via the input unit 417, the prescription control unit 562 determines to execute the tele-prescription and the processing proceeds to step S412.

In step S412, the pharmacy cart 16 executes tele-prescription execution processing. Thereafter, the processing returns to step S402 and processing subsequent to step S402 is performed.

Here, details of the tele-prescription execution processing will be described with reference to the flowcharts of Figs. 20 and 21.

In step S451, the prescription control unit 562 makes a request for supplying the tele-prescription. Specifically, the prescription control unit 562 generates the above-described tele-prescription request information. The communication unit 419 transmits the tele-prescription request information to the server 11 via the network 31 under the control of the communication control unit 565.

In step S452, the prescription control unit 562 permits access to the medical information of the patient of the patient DB 12 in the same processing as that of step S252 of the medical cart 15 in Fig. 16.

In step S453, the prescription control unit 562 determines whether the pharmacist is introduced. When it is determined that the pharmacist is not instructed, the processing proceeds to step S454.

In step S454, the prescription control unit 562 determines whether an alternative is proposed. When it is determined that the alternative is not proposed, the processing returns to step S453.

Thereafter, until it is determined in step S453 that the pharmacist is introduced or it is determined in step S454 that the alternative is proposed, the processing of steps S453 and S454 is repeatedly performed.

Conversely, when the alternative information transmitted by the server 11 in the processing of step S156 of Fig. 13, as described above, is received via the network 31 and the communication unit 419 in step S454, the prescription control unit 562 determines that the alternative is proposed and the processing proceeds to step S455.

In step S455, the pharmacy cart 16 proposes the alternative. Specifically, the output unit 418 outputs at least one of visual information and auditory information for notifying the alternative indicated by the alternative information under the control of the output control unit 564. For example, a screen on which the alternative is proposed is displayed on the output unit 418 or a sound proposing the alternative is output from the output unit 418.

Thereafter, the processing proceeds to step S460.

Conversely, when the pharmacist introduction information transmitted from the server 11 via the network 31 and the communication unit 419 in step S154 of Fig. 13, as described above, is received in step S453, the prescription control unit 562 determines that the pharmacist is introduced and the processing proceeds to step S456.

In step S456, the pharmacy cart 16 proposes information regarding the introduced pharmacist. For example, the output unit 418 displays a screen on which the information regarding the pharmacist indicated in the pharmacist introduction information is shown under the control of the output control unit 564.

In step S457, the prescription control unit 562 determines whether the introduced pharmacist is selected. When it is determined that the introduced pharmacist is not selected, the processing proceeds to step S458.

In step S458, the prescription control unit 562 determines whether the introduction of another pharmacist is requested. For example, when the patient performs an operation of requesting the introduction of the other pharmacist via the input unit 417, the prescription control unit 562 determines that the introduction of the other pharmacist is requested and the processing proceeds to step S459.

In step S459, the pharmacy cart 16 makes a request for introducing the other pharmacist. Specifically, the prescription control unit 562 generates the above-described pharmacist introduction request information. The communication unit 419 transmits the pharmacist introduction request information to the server 11 via the network 31 under the control of the communication control unit 565.

Thereafter, the processing returns to step S453 and the processing subsequent to step S453 is performed.

Conversely, when the patient does not perform the operation of requesting the introduction of the other pharmacist in step S458, the prescription control unit 562 determines that the introduction of the other pharmacist is not requested and the processing proceeds to step S460. This is a case in which the introduced pharmacist is not selected and the introduction of the other pharmacist is not requested.

In step S460, the prescription control unit 562 prohibits access to the medical information of the patient of the patient DB 12 in the same processing as that of step S260 of the medical cart 15 in Fig. 16.

Thereafter, the tele-prescription execution processing ends.

Conversely, for example, when the patient performs the operation of selecting the introduced pharmacist via the input unit 417 in step S457, the prescription control unit 562 determines that the introduced pharmacist is selected and the processing proceeds to step S461.

Thus, for example, when one pharmacist is introduced and the introduced pharmacist is selected and two or more pharmacists are introduced, and a desired pharmacist is selected from them.

In step S461, the pharmacy cart 16 starts communication with the selected pharmacist. Specifically, the communication unit 419 starts communication with the medical practitioner terminal 14 of the selected pharmacist via the network 31 under the control of the communication control unit 565.

For example, the communication unit 419 starts transmitting an image and a sound of the patient to the medical practitioner terminal 14 under the control of the communication control unit 565 and starts receiving an image and a sound of the pharmacist from the medical practitioner terminal 14. Thus, the patient and the pharmacist start telecommunication using the mutual images and sounds.

In step S462, the prescription control unit 562 determines whether telemanipulation data of the prescription device is received.

For example, when the pharmacist performs telemanipulation on the prescription device included in the prescription processing unit 511 of the pharmacy cart 16, the pharmacist inputs an instruction of the telemanipulation of the prescription device to the medical practitioner terminal 14. The medical practitioner terminal 14 generates telemanipulation data indicating manipulation content in the telemanipulation in step S503 of Fig. 22 to be described below and transmits the telemanipulation data to the pharmacy cart 16.

When the prescription control unit 562 determines that the telemanipulation data is received from the medical practitioner terminal 14 via the network 31 and the communication unit 419, the processing proceeds to step S463.

In step S463, the prescription control unit 562 controls an operation of the prescription device included in the prescription processing unit 511 based on the telemanipulation data.

Thereafter, the processing proceeds to step S464.

Conversely, when it is determined in step S462 that the telemanipulation data of the prescription device is not received, the processing of step S463 is skipped and the processing proceeds to step S464.

In step S464, the prescription control unit 562 determines whether the tele-prescription ends. When it is determined that the tele-prescription does not end, the processing returns to step S462.

Thereafter, until it is determined in step S464 that the tele-prescription ends, the processing of steps S462 to S464 is repeatedly performed.

Thus, for example, the pharmacist remotely dispenses medicine based on the prescription data of the patient and supplies the dispensed medicine to the patient while remotely manipulating the prescription device of the pharmacy cart 16, as necessary, using the medical practitioner terminal 14. For example, the pharmacist responds to consultation of the patient or gives a medication instruction as necessary.

Conversely, for example, when the patient performs a manipulation to end the tele-prescription via the input unit 417 in step S464, the prescription control unit 562 determines that the tele-prescription ends. The prescription control unit 562 generates tele-prescription execution end information to notify of end of the tele-prescription. The communication unit 419 transmits the tele-prescription execution end information to the medical practitioner terminal 14 of the medical practitioner via the network 31 under the control of the communication control unit 565. Thereafter, the processing proceeds to step S465.

Conversely, for example, when the pharmacist inputs an instruction to end the supply of the tele-prescription to the medical practitioner terminal 14 in step S504 of Fig. 22 to be described below, the medical practitioner terminal 14 generates tele-prescription supply end information to notify of end of the supply of the tele-prescription and transmits the tele-prescription supply end information to the pharmacy cart 16.

When the tele-prescription supply end information is received from the medical practitioner terminal 14 via the network 31 and the communication unit 419 in step S464, the prescription control unit 562 determines that the tele-prescription ends and the processing proceeds to step S465.

In step S465, the pharmacy cart 16 ends the communication with the pharmacist. That is, the communication unit 419 disconnects the communication with the medical practitioner terminal 14 of the pharmacist executing the tele-prescription under the control of the communication control unit 565.

In step S466, the access to the medical information of the patient is prohibited as in the processing of step S460.

Thereafter, the tele-prescription execution processing ends.

Referring back to Fig. 19, conversely, when it is determined in step S411 that the tele-prescription is not executed, the processing returns to step S402 and the processing subsequent to step S402 is performed.

### <Tele-Prescription Control Processing>

Next, tele-prescription control processing performed by the medical practitioner terminal 14 of the pharmacist to correspond to the tele-prescription execution processing of the pharmacy cart 16 in Figs. 20 and 21 will be described with reference to the flowchart of Fig. 22.

In step S501, the medical practitioner terminal 14 starts communication with the patient. Specifically, the communication unit 218 starts communication with the pharmacy cart 16 used by the patient via the network 31 to correspond to the processing of the pharmacy cart 16 of step S261 of Fig. 20, as described above.

In step S502, the processor 211 determines whether the telemanipulation is performed on the prescription device of the pharmacy cart 16.

For example, when the pharmacist performs a telemanipulation of the prescription device included in the prescription processing unit 511 of the pharmacy cart 16, the pharmacist inputs an instruction of the tele-prescription via the input unit 216. When an input of the instruction of the telemanipulation is detected, the processor 211 determines that the telemanipulation of the prescription device of the pharmacy cart 16 is performed and the processing proceeds to step S503.

In step S503, the medical practitioner terminal 14 transmits telemanipulation data of the prescription device of the pharmacy cart 16. Specifically, the processor 211 generates the above-described telemanipulation data and transmits the telemanipulation data to the pharmacy cart 16 via the communication unit 218 and the network 31.

Thereafter, the processing proceeds to step S504.

Conversely, when it is determined in step S502 that the telemanipulation is not performed on the prescription device of the pharmacy cart 16, the processing of step S503 is skipped and the processing proceeds to step S504.

In step S504, the processor 211 determines whether the tele-prescription ends. When it is determined that the tele-prescription does not end, the processing returns to step S502.

Thereafter, until it is determined in step S504 that the tele-prescription ends, the processing of steps S502 to S504 is repeatedly performed.

Conversely, for example, when the pharmacist performs a manipulation to end of the tele-prescription via the input unit 216 in step S504, the processor 211 determines that the tele-prescription ends. The processor 211 generates the tele-prescription supply end information to notify of end of the tele-prescription and transmits the tele-prescription supply end information to the pharmacy cart 16 of the patient via the communication unit 218 and the network 31. Thereafter, the processing proceeds to step S309.

For example, when the tele-prescription execution end information transmitted by the pharmacy cart 16 in the processing of step S464 of Fig. 21, as described above, is received via the network 31 and the communication unit 218, the processor 211 determines that the tele-prescription ends and the processing proceeds to step S505.

In step S505, the medical practitioner terminal 14 ends the communication with the patient. That is, the communication unit 218 disconnects the communication with the pharmacy cart 16 of the patient supplying the tele-prescription.

Thereafter, the tele-prescription control processing ends.

In this way, the patient can receive suitable telemedicine from a suitable medical practitioner or receive suitable tele-prescription from a suitable pharmacist.

The patient can receive the telemedicine and the tele-prescription without preparing for a special device or the like. Further, since the medical cart 15 can be equipped with an advanced medical device better than the home or the like of the patient, the patient can receive more advanced telemedicine (for example, image analysis, precise measurement, or the like).

Further, the medical cart 15 and the pharmacy cart 16 are moved to the vicinity of the patient. Thus, although the patient goes to a distant place, the patient can receive a medical service or a prescription service. For example, even a patient who is difficult to move can easily receive a medical service or a prescription service.

When the patient wants to receive the telemedicine or the tele-prescription, applicable medical practitioners or pharmacists can be searched for and introduced. Therefore, it is possible to shorten a waiting time of the patient.

Further, since a private space is guaranteed inside the medical cart 15, the patient can receive the telemedicine or the tele-prescription without feeling hesitant. The privacy of the patient can be ensured.

### «2. Modified Examples»

Hereinafter, modified examples of the above-described embodiments of the present technology will be described.

### <Modified Examples of Method of Matching Patients with Medical Practitioners and Pharmacist>

The above-described method of matching patients with medical practitioners and pharmacists is exemplary and can be modified.

Fig. 23 is a flowchart illustrating a modified example of the medical practitioner matching processing in step S9 of Fig. 10.

In step S601, the medical information of the patient is acquired as in the processing of step S101 of Fig. 12, as described above.

In step S602, the state of the patient is analyzed as in the processing of step S102 of Fig. 12, as described above.

In step S603, the analysis unit 161 determines whether supply of the telemedicine is possible. For example, when the telemedicine action necessary for the patient can be executed using the medical processing unit 422 of the attention medical cart 15 and the telemedicine action is not against the wishes of the patient, the analysis unit 161 determines that the supply of the telemedicine is possible and the processing proceeds to step S604.

In step S604, the server 11 sounds the supply of the telemedicine to each medical practitioner. Specifically, the selection unit 162 generates telemedicine supply sounding information to sound charge of the telemedicine to each medical practitioner and transmits the telemedicine supply sounding information to the medical practitioner terminal 14 of each medical practitioner via the network 31.

The telemedicine supply sounding information includes, for example, information similar to the above-described telemedicine request information.

On the other hand, for example, when the supply of the telemedicine is possible, each medical practitioner inputs the possibility of the supply of the telemedicine to the medical practitioner terminal 14. The medical practitioner terminal 14 generates the telemedicine supply possibility information indicating that the supply of the telemedicine is possible and transmits the telemedicine supply possibility information to the server 11.

The selection unit 162 receives the telemedicine supply possibility information from the medical practitioner terminal 14 of each medical practitioner capable of supplying the telemedicine via the network 31 and the communication unit 118.

The telemedicine supply possibility information includes, for example, information regarding a facial photo, a profile, a field of specialization, a career, or the like of the medical practitioner.

In step S605, the selection unit 162 selects the medical practitioner to be introduced from the responding medical practitioners. For example, the selection unit 162 selects a predetermined number of medical practitioners in order in which the matching ratio with the desired condition of the patient is higher among the medical practitioners who respond in the processing of step S604 as the medical practitioners introduced to the patient.

In step S606, the selection unit 162 determines whether supply of the telemedicine is possible. When there is the medical practitioner who can be introduced to the patient, the selection unit 162 determines that the supply of the telemedicine is possible and the processing proceeds to step S607.

In step S607, the medical practitioner is introduced as in the processing of step S105 of Fig. 12.

In step S608, it is determined whether the introduction of other medical practitioners is requested as in the processing of step S608 of Fig. 12. When it is determined that the introduction of the other medical practitioners is requested, the processing returns to step S602.

Thereafter, the processing of steps S602 to S608 is repeatedly performed until it is determined in step S603 or S606 that the supply of the telemedicine is not possible or it is determined in step S608 that the introduction of the other medical practitioners is not requested.

Conversely, when it is determined in step S608 that the introduction of the other medical practitioners is not requested, the medical practitioner matching processing ends.

When there is no medical practitioner who can be introduced to the patient in step S606, it is determined that the supply of the telemedicine is not possible and the processing proceeds to step S609.

Further, when the telemedicine action necessary for the patient cannot be executed using the medical processing unit 422 of the attention medical cart 15 in step S603 or the telemedicine action is against the wish of the patient, it is determined that the supply of the telemedicine is not possible, and the processing proceeds to step S609.

In step S609, an alternative is proposed as in the processing of step S107 of Fig. 12.

Thereafter, the medical practitioner matching processing ends.

In this way, it is sounded to each medical practitioner whether the supply of the telemedicine to the patient is possible and a responding medical practitioner can be introduced to the patient.

For example, the responding medical practitioner may be introduced to the patient in the order of the response.

Fig. 24 is a flowchart illustrating a modified example of the pharmacist matching processing of step S11 of Fig. 10.

In step S651, the medical information of the patient is acquired as in the processing of step S151 of Fig. 13, as described above.

In step S652, the server 11 sounds the supply of the tele-prescription to each pharmacist. Specifically, the selection unit 162 generates tele-prescription supply sounding information to sound charge of the tele-prescription to each pharmacist and transmits the tele-prescription supply sounding information to the medical practitioner terminal 14 of each pharmacist via the network 31.

The tele-prescription supply sounding information includes, for example, information similar to the above-described tele-prescription request information.

On the other hand, for example, when the supply of the tele-prescription is possible, each pharmacist inputs the possibility of the supply of the tele-prescription to the medical practitioner terminal 14. The medical practitioner terminal 14 generates the tele-prescription supply possibility information indicating that the supply of the tele-prescription is possible and transmits the tele-prescription supply possibility information to the server 11.

The selection unit 162 receives the tele-prescription supply possibility information from the medical practitioner terminal 14 of each pharmacist capable of supplying the tele-prescription via the network 31 and the communication unit 118.

The tele-prescription supply possibility information includes, for example, information regarding a facial photo, a profile, a field of specialization, a career, or the like of the pharmacist.

In step S653, the selection unit 162 selects the pharmacist to be introduced from the responding pharmacist. For example, the selection unit 162 selects a predetermined number of pharmacists in order in which the matching ratio with the desired condition of the patient is higher among the pharmacists who respond in the processing of step S652 as the pharmacist introduced to the patient.

In step S654, the selection unit 162 determines whether supply of the tele-prescription is possible. When there is the pharmacist who can be introduced to the patient, the selection unit 162 determines that the supply of the tele-prescription is possible and the processing proceeds to step S655.

In step S655, the pharmacist is introduced as in the processing of step S154 of Fig. 13.

In step S656, it is determined whether the introduction of other pharmacists is requested as in the processing of step S155 of Fig. 13. When it is determined that the introduction of the other pharmacists is requested, the processing returns to step S652.

Thereafter, the processing of steps S652 to S656 is repeatedly performed until it is determined in step S654 that the supply of the tele-prescription is possible or it is determined in step S656 that the introduction of the other pharmacists is not requested.

Conversely, when it is determined in step S656 that the introduction of the other pharmacists is not requested, the pharmacist matching processing ends.

When there is no medical practitioner who can be introduced to the patient in step S654, it is determined that the supply of the tele-prescription is not possible and the processing proceeds to step S657.

In step S657, an alternative is proposed as in the processing of step S156 of Fig. 13.

Thereafter, the pharmacist matching processing ends.

In this way, it is sounded to each pharmacist whether the supply of the telemedicine to the patient is possible and a responding pharmacist can be introduced to the patient.

For example, the responding pharmacist may be introduced to the patient in the order of the response.

For example, the selection unit 162 of the server 11 may collect assessment of each medical practitioner by each patient and present the collected assessment as one selection condition of the medical practitioner to the patient.

Further, for example, the patient may select a plurality of medical practitioners and receive the telemedicine from the plurality of medical practitioners. Thus, for example, the patient can receive opinions about examination content, a curing method, or the like from the plurality of medical practitioners.

For example, when the patient receives an examination of the telemedicine at the second or subsequent time, the medical practitioner who has previously executed the telemedicine on the patient may be preferentially introduced to the patient. Similarly, for example, when the patient receives the tele-prescription at the second or subsequent time, the pharmacist who has previously executed the tele-prescription on the patient may be preferentially introduced to the patient.

Further, for example, a method of selecting the introduced medical practitioner may be changed in accordance with a symptom of the patient. For example, when the patient suffers from a mental disease, the previous same medical practitioner may be preferentially introduced. For example, when the patient suffers from a slight illness such as a cold, the medical practitioner who can supply the telemedicine as quickly as possible may be preferentially introduced.

For example, when a patient who comes from a foreign country has subscribed in a medical insurance for trip, a medical practitioner to whom the medical insurance can be applied may be preferentially introduced to the patient.

Further, a telemedical fee of each medical practitioner may be variable. For example, the telemedical fee of a medical practitioner who is popular or a medical practitioner who has high assessment may be set to be high.

For example, a telemedical fee in a busy period of time or a crowded period of time may be set to be high and a telemedical fee in an off-time or a free time may be set to be low. For example, in a crowded period of time, the telemedicine may be preferentially supplied to a patient who pays a higher medical fee.

For example, a pharmacist may be introduced to a patient based on only whether the tele-prescription can be supplied at a desired time without accepting a desired condition of the patient.

Further, for example, congeniality between a patient and a medical practitioner or a pharmacist may be estimated and the medical practitioner or the pharmacist may be introduced to the patient based on the result.

For example, when a time of the telemedicine becomes long, a medical practitioner who has a fee time may be preferentially introduced to an assumed patient (for example, a patient who receives an examination of a mental disease).

### <Modified Examples of Medical Cart 15 and Pharmacy Cart 16>

For example, the telemedicine may be executed during movement of the medical cart 15. For example, the patient may receive the telemedicine while the medical cart 15 is transporting the patient to a destination (for example, a medical organization, his or her home, or the like).

For example, a kind of available telemedicine may be changed during stopping or movement of the medical cart 15. For example, measurement of biological information of a medical inquiry, pulses, or the like may be performed during the stopping of the medical cart 15 and an examination of a mental disease may be performed during the movement.

Further, not only the inside but also the outside of the medical cart 15 may be effectively utilized.

For example, a patient may be received outside of the medical cart 15.

For example, when a medical practitioner executes a simple examination on the patient remotely using images of the external cameras 312F to 312B of the medical cart 15 and determines that further examination is necessary, the patient may be guided into the cart to perform more advanced telemedicine.

For example, a life-sized trainer may be displayed on the display unit 311F of the medical cart 15 to guide a healthy check or an exercise.

Not only the inside but also the outside of the pharmacy cart 16 may be effectively utilized.

For example, an external display unit of the pharmacy cart 16 may be used to explain a dispensed medicine. Thus, it is possible to shorten a time in which a patient stays inside the pharmacy cart 16 and it is possible to shorten waiting times of other patients.

Further, for example, a sellable medicine may be displayed on an external display unit of the pharmacy cart 16 and medicine selling may be performed.

For example, a nurse may reside in each medical cart 15 to assist the telemedicine in the medical cart 15.

Further, for example, a virtual medical practitioner appears within a field of view of a patient inside the medical cart 15 to execute the telemedicine in accordance with a virtual reality (VR) technology. Similarly, a virtual prescriptionist may appear within a field of view of a patient inside the pharmacy cart 16 to execute the tele-prescription.

The vehicle 301 configuring the medical cart 15 and the pharmacy cart 16 is exemplary and another type of vehicle may be used.

For example, Fig. 25 illustrates an exemplary configuration of the exterior appearance of a vehicle 601 which can be used as the medical cart 15 and the pharmacy cart 16. Fig. 25 illustrates an exemplary configuration of an exterior appearance when the vehicle 601 is viewed diagonally in front and to the left.

A display unit 611F, a display unit 611L, a display unit 611R (not illustrated), and a display unit 611B (not illustrated) are provided on the front surface, the left side surface, the right side surface, and the back surface of the vehicle 601, respectively, as in the vehicle 301 of Fig. 5.

A camera 612F, a camera 612L, a camera 612R (not illustrated), and a camera 612B (not illustrated) are provided on the front surface, the left side surface, the right side surface, and the back surface of the vehicle 601 as in the vehicle 301.

The vehicle 601 is different from the vehicle 301 in that the vehicle 601 has a completely closed space inside. Thus, privacy or security of a patient can be guaranteed more rigidly and a user can use the vehicle 601 (the medical cart and the pharmacy cart) more at ease.

A moving object providing the telemedicine and the tele-prescription is not limited to the above-described cart type of vehicle, but another type of vehicle can also be applied.

Further, a moving object providing the telemedicine and the tele-prescription is not limited to the vehicle, but another kind of moving object may be used.

A moving object providing the telemedicine and the tele-prescription may be a type of moving object which a driver drives.

### <Modified Examples of Virus Countermeasures>

For example, the medical processing unit 422 of the medical cart 15 may include an ultraviolet irradiation unit. For example, after a patient gets off the medical cart 15, the ultraviolet irradiation unit irradiates the inside and the surroundings of the medical cart 15 with an ultraviolet ray to remove various viruses. Thus, secondary infection of various infectious diseases in the medical cart 15 can be prevented.

For example, the medical processing unit 422 may include an antiseptic solution spraying unit that sprays an antiseptic solution to the surroundings of the medical cart 15. For example, the antiseptic solution spraying unit may spray an antiseptic solution to the surroundings of the medical cart 15 in a broad space or the like where there are no surrounding people. Thus, for example, unmanned antisepsis can be performed using an antiseptic solution with high concentration.

Similarly, for example, the prescription processing unit 511 of the pharmacy cart 16 may include an ultraviolet irradiation unit and an antiseptic solution spraying unit to perform similar processing.

For example, the sensor group 420 of the medical cart 15 may include an infrared sensor. The infrared sensor may measure a body temperature of a patient outside of the medical cart 15. Similarly, for example, the sensor group 420 of the pharmacy cart 16 may include an infrared sensor. The infrared sensor may measure a body temperature of a patient outside of the pharmacy cart 16.

For example, the infrared sensor of each medical cart 15 and each pharmacy cart 16 may measure body temperatures of many and unspecified surrounding people. Thus, for example, body temperatures of surrounding people can be collectively detected in a place such as a station where people gather. For example, the observation unit 461 of each medical cart 15 and the observation unit 561 of each pharmacy cart 16 may detect abnormal people who are suspicious of virus infection or the like based on measurement results of the body temperatures of surrounding people.

On the other hand, for example, the analysis unit 161 of the server 11 may receive detection results of abnormal people from each medical cart 15 and each pharmacy cart 16 and construct a database indicating the detection results of the abnormal people (for example, a distribution or transition of the abnormal people) at each district.

For example, when patients are infected with predetermined viruses, the communication unit 419 of the medical cart 15 may communicate with the patient terminal 17 of the patients (hereinafter referred to as infected people) under the communication control unit 465 and may acquire behavior history data indicating a behavior history of infected people. For example, the behavior history data may include a schedule and a movement route of past infected people.

On the other hand, for example, the organization unit 164 of the server 11 may receive the behavior history data of the infected people from the medical cart 15 and may set a disposition place or a patrol route of each medical cart 15 and each pharmacy cart 16 based on the behavior history data of the infected people. For example, the organization unit 164 may specify a focused medical district based on the behavior history data of the infected people and may cause dispatching or patrolling to be performed by preferring the medical cart 15 and the pharmacy cart 16 in the focused medical district. The focused medical district includes, for example, a district where there is concern of a cluster of an infectious disease occurring or a district where there is a possibility of close contact people with infected people.

For example, the server 11 may receive the behavior history data from the patient terminal 17 of an infected person without being involved with the medical cart 15.

For example, the observation unit 461 of the medical cart 15 may specify a focused medical district based on the behavior history data of the infected people, and the movement control unit 463 may set a destination or a route based on the focused medical district and control movement of the medical cart 15.

For example, the observation unit 461 of the medical cart 15 may detect the number of people and a ratio at which people wear masks around the medical cart 15 by performing image recognition of surrounding images obtained by imaging the surroundings of the medical cart 15. Similarly, the observation unit 561 of the pharmacy cart 16 may detect the number of people and a ratio at which people wear masks around the pharmacy cart 16 by performing image recognition of surrounding images obtained by imaging the surroundings of the pharmacy cart 16.

Then, the analysis unit 161 of the server 11 may receive detection results of the number of people and the ratios at which people wear masks around each medical cart 15 and each pharmacy cart 16, estimate a population density and a ratio at which people wear masks in each district based on the received detection results, and generate an infection risk map indicating an infection risk of a predetermined virus. For example, the organization unit 164 of the server 11 may set a disposition place or a patrol route of each medical cart 15 and each pharmacy cart 16 based on the infection risk map. For example, the organization unit 164 may preferentially dispose or tour the medical cart 15 and the pharmacy cart 16 in a district where an infection risk is high.

For example, the observation unit 461 of each medical cart 15 may generate a surrounding infection risk map based on the number of surrounding people and a mask wearing ratio. The movement control unit 463 of each medical cart 15 may set a destination or a route based on the surrounding infection risk map and control the movement of the medical cart 15.

Similarly, for example, the observation unit 561 of each pharmacy cart 16 may generate a surrounding infection risk map based on the number of surrounding people and a mask wearing ratio. The movement control unit 563 of each pharmacy cart 16 may set a destination or a route based on the surrounding infection risk map and control the movement of the pharmacy cart 16.

### <Modified Examples of Configuration of Telemedicine System 1>

The configuration of the above-described telemedicine system 1 is exemplary and can be modified.

For example, some of the processing of the server 11 may be performed by the medical practitioner terminal 14, the medical cart 15, or the pharmacy cart 16.

For example, the processing of the selection unit 162 of the server 11 may be performed by the medical cart 15 and the pharmacy cart 16. For example, the medical cart 15 may select a medical practitioner to be introduced to a patient based one or more of a patient state, a desired condition, and the like. Similarly, the pharmacy cart 16 may select a pharmacist to be introduced to a patient based one or more of a patient state, a desired condition, and the like.

For example, the medical cart 15 may analyze a patient state and select an appropriate telemedical action or may determine whether the supply of the appropriate telemedical action is possible.

For example, each medical cart 15 and each pharmacy cart 16 may communicate with each other and may autonomously set each disposition place or route while having cooperation. For example, the pharmacy cart 16 founds a timing at which the telemedicine of the medical cart 15 ends and moves to the vicinity of the medical cart 15.

Further, each medical cart 15 and each pharmacy cart 16 may predict a patient demand.

The division into the medical cart 15 and pharmacy cart 16 is not necessarily required and the telemedicine and the tele-prescription may be executed in one cart.

### <Other Modified Examples>

In the foregoing description, the example in which the patient DB 12 stores a clinical card of a patient has been described. For example, a patient may store an own clinical card on the patient terminal 17 to carry the patient terminal 17. In this case, for example, when the patient is inside the medical cart 15 or the pharmacy cart 16, the clinical card of the patient of the patient terminal 17 may be in an accessible state. Thus, the medical practitioner or the pharmacist can browse the clinical card of a patient only when the patient is inside the medical cart 15 or the pharmacy cart 16, and thus personal information of the patient is protected.

For example, when a person estimated to wander due to cognitive impairment, a person estimated to feel sick, or the like is detected, the medical cart 15 may say something to the person.

Further, for example, a demand of the pharmacy cart 16 may be predicted based on an expiration date of distributed medicine or the like.

### «3. Others»

The above-described series of steps of processing may be performed by hardware or software. When the series of steps of processing is performed by software, a program of the software is installed in a computer. Here, the computer includes a computer embedded in dedicated hardware or, for example, a general-purpose computer capable of executing various functions by installing various programs.

A program executed by a computer may be recorded on, for example, a removable medium (for example, the recording medium 114 of Fig. 2, the recording medium 214 of Fig. 4, the recording medium 414 of Fig. 6, or the recording medium 414 of Fig. 8) which is a package medium for supply. The program can be supplied via a wired or wireless transfer medium such as a local area network, the Internet, or digital satellite broadcasting.

A program executed by a computer may be a program which performs steps of processing chronologically in a sequence described in the present specification or may be a program that performs steps of processing in parallel or at a necessary timing such as a calling.

In the present specification, the system means a set of a plurality of constituent elements (devices, modules (components), or the like) and all the constituent elements may be included or not included in the same casing. Accordingly, a plurality of devices accommodated in separate casings and connected via a network and one device in which a plurality of modules are accommodated in one casing may all be a system.

Embodiments of the present technology are not limited to the above-described embodiments and can be modified in various forms within the scope of the present technology departing from the gist of the present technology.

For example, the present technology may have a configuration of clouding computing in which a plurality of devices share and process one function together via a network.

The steps described in the above-described flowchart can be execute by one device or may be shared and performed by a plurality of devices.

Further, when a plurality of steps of processing are included in one step, the plurality of steps of processing included in the one step may be performed by one device or may be shared and performed by a plurality of devices.

### <Combination Examples of Configurations>

The present technology can be configured as follows.

(1) A moving object including:
   a movement control unit configured to control movement based on a patient demand;
   a communication control unit configured to control communication with a medical practitioner terminal which is an information processing terminal of a medical practitioner executing telemedicine; and
   a medical control unit configured to control the telemedicine with the medical practitioner terminal.
(2) The moving object according to (1), further including:
   an output control unit configured to control outputting of information regarding medical practitioners introduced to the patient,
   wherein the communication control unit controls communication with the medical practitioner terminal of a medical practitioner selected among the introduced medical practitioners.
(3) The moving object according to (2), wherein the communication control unit controls reception of the information regarding the medical practitioners from an information processing device selecting the medical practitioners introduced to the patient based on at least one of a patient state and a desired condition.
(4) The moving object according to (1), further including:
   a selection unit configured to select a medical practitioner based on at least one of a patient state and a desired condition.
(5) The moving object according to any one of (1) to (4), wherein the movement control unit controls movement to a destination designated based on the demand of the patient.
(6) The moving object according to any one of (1) to (5), the movement control unit controls movement of a route designated based on the demand of the patient.
(7) The moving object according to any one of (1) to (6),
   wherein the communication control unit controls reception of behavior history data of the patient from a patient terminal which is an information processing terminal of the patient, and
   wherein the movement control unit controls the movement based on the behavior history data.
(8) The moving object according to any one of (1) to (7), further including:
   an observation unit configured to detect the demand of the patient by observing surroundings,
   wherein the movement control unit controls the movement based on the detected demand of the patient.
(9) The moving object according to (8),
   wherein the observation unit detects a person who is a telemedicine execution target, and
   wherein the movement control unit performs control such that the moving object is stopped near the detected person.
(10) The moving object according to any one of (1) to (9), further including:
   a medical device used for telemedicine,
   wherein the medical control unit controls the medical device based on telemanipulation data from the medical practitioner terminal.
(11) The moving object according to any one of (1) to (10), wherein the medical control unit performs access control to medical information of a patient receiving the telemedicine.
(12) The moving object according to any one of (1) to (11), further including:
   a prescription device used for tele-prescription; and
   a prescription control unit configured to control the prescription device based on telemanipulation data from the medical practitioner terminal.
(13) An information processing method causing a moving object to perform:
   controlling movement based on a patient demand;
   controlling communication with a medical practitioner terminal which is an information processing terminal of a medical practitioner executing telemedicine; and
   controlling the telemedicine with the medical practitioner terminal.
(14) An information processing device including:
   a selection unit configured to select a medical practitioner introduced to a patient; and
   a communication control unit configured to control transmission of information regarding the medical practitioner introduced to the patient to a moving object used by the patient.
(15) The information processing device according to (14), wherein the selection unit selects the medical practitioner introduced to the patient based on at least one of a patient state and a desired condition.
(16) The information processing device according to (14) or (15),
   wherein the selection unit selects the medical practitioner executing the telemedicine for the patient, and
   wherein the communication control unit controls transmission of information regarding the medical practitioner to the moving object in which the patient receives the telemedicine.
(17) The information processing device according to any one of (14) to (16),
   wherein the selection unit selects a pharmacist who is a medical practitioner executing the tele-prescription for the patient, and
   wherein the communication control unit controls transmission of information regarding the medical practitioner to the moving object in which the patient receives the tele-prescription.
(18) The information processing device according to any one of (14) to (17),
   an organization unit configured to set a destination or a route to the moving object,
   wherein the communication control unit controls transmission of information regarding the destination or the route of the moving object to the moving object.
(19) The information processing device according to (18), further including:
   a demand prediction unit configured to predict a demand of the patient,
   wherein the organization unit sets the destination or the route of the moving object based on the predicted demand of the patient.
(20) The information processing device according to (18) or (19),
   wherein the communication control unit controls reception of behavior history data of the patient from the moving object or a patient terminal which is an information processing terminal of the patient, and
   wherein the organization unit sets the destination or the route of the moving object based on the behavior history data.

The advantageous effects described in the present specification are merely exemplary and are not limited, and other advantageous effects may be obtained.

### [Reference Signs List]

- 1: Telemedicine system
- 11: Server
- 12: Patient DB
- 13: Medical DB
- 14-1 to 14-m: Medical practitioner terminal
- 15-1 to 15-n: Medical cart
- 16-1 to 16-p: Pharmacy cart
- 17-1 to 17-q: Patient terminal
- 111: Processor
- 118: Communication unit
- 151: Information processing unit
- 161: Analysis unit
- 162: Selection unit
- 163: Demand prediction unit
- 164: Organization unit
- 201: Information processing terminal
- 211: Processor
- 218: Communication unit
- 301: Vehicle
- 411: Processor
- 415: Driving unit
- 418: Output unit
- 419: Communication unit
- 420: Sensor group
- 422: Medical processing unit
- 451: Information processing unit
- 461: Observation unit
- 462: Medical control unit
- 463: Movement control unit
- 464: Output control unit
- 465: Communication control unit
- 511: Prescription processing unit
- 551: Information processing unit
- 561: Observation unit
- 562: Prescription control unit
- 563: Movement control unit
- 564: Output control unit
- 565: Communication control unit

## Claims

1. A moving object comprising:
a movement control unit configured to control movement based on a patient demand;
a communication control unit configured to control communication with a medical practitioner terminal which is an information processing terminal of a medical practitioner executing telemedicine; and
a medical control unit configured to control the telemedicine with the medical practitioner terminal.

2. The moving object according to claim 1, further comprising:
an output control unit configured to control outputting of information regarding medical practitioners introduced to the patient,
wherein the communication control unit controls communication with the medical practitioner terminal of a medical practitioner selected among the introduced medical practitioners.

3. The moving object according to claim 2, wherein the communication control unit controls reception of the information regarding the medical practitioners from an information processing device selecting the medical practitioners introduced to the patient based on at least one of a patient state and a desired condition.

4. The moving object according to claim 1, further comprising: a selection unit configured to select a medical practitioner based on at least one of a patient state and a desired condition.

5. The moving object according to claim 1, wherein the movement control unit controls movement to a destination designated based on the demand of the patient.

6. The moving object according to claim 1, wherein the movement control unit controls movement of a route designated based on the demand of the patient.

7. The moving object according to claim 1,
wherein the communication control unit controls reception of behavior history data of the patient from a patient terminal which is an information processing terminal of the patient, and
wherein the movement control unit controls the movement based on the behavior history data.

8. The moving object according to claim 1, further comprising:
an observation unit configured to detect the demand of the patient by observing surroundings,
wherein the movement control unit controls the movement based on the detected demand of the patient.

9. The moving object according to claim 8,
wherein the observation unit detects a person who is a telemedicine execution target, and
wherein the movement control unit performs control such that the moving object is stopped near the detected person.

10. The moving object according to claim 1, further comprising:
a medical device used for telemedicine,
wherein the medical control unit controls the medical device based on telemanipulation data from the medical practitioner terminal.

11. The moving object according to claim 1, wherein the medical control unit performs access control to medical information of a patient receiving the telemedicine.

12. The moving object according to claim 1, further comprising:
a prescription device used for tele-prescription; and
a prescription control unit configured to control the prescription device based on telemanipulation data from the medical practitioner terminal.

13. An information processing method causing a moving object to perform:
controlling movement based on a patient demand;
controlling communication with a medical practitioner terminal which is an information processing terminal of a medical practitioner executing telemedicine; and
controlling the telemedicine with the medical practitioner terminal.

14. An information processing device comprising:
a selection unit configured to select a medical practitioner introduced to a patient; and
a communication control unit configured to control transmission of information regarding the medical practitioner introduced to the patient to a moving object used by the patient.

15. The information processing device according to claim 14, wherein the selection unit selects the medical practitioner introduced to the patient based on at least one of a patient state and a desired condition.

16. The information processing device according to claim 14,
wherein the selection unit selects the medical practitioner executing the telemedicine for the patient, and
wherein the communication control unit controls transmission of information regarding the medical practitioner to the moving object in which the patient receives the telemedicine.

17. The information processing device according to claim 14,
wherein the selection unit selects a pharmacist who is a medical practitioner executing the tele-prescription for the patient, and
wherein the communication control unit controls transmission of information regarding the medical practitioner to the moving object in which the patient receives the tele-prescription.

18. The information processing device according to claim 14,
an organization unit configured to set a destination or a route to the moving object,
wherein the communication control unit controls transmission of information regarding the destination or the route of the moving object to the moving object.

19. The information processing device according to claim 18, further comprising:
a demand prediction unit configured to predict a demand of the patient,
wherein the organization unit sets the destination or the route of the moving object based on the predicted demand of the patient.

20. The information processing device according to claim 18,
wherein the communication control unit controls reception of behavior history data of the patient from the moving object or a patient terminal which is an information processing terminal of the patient, and
wherein the organization unit sets the destination or the route of the moving object based on the behavior history data.
